(19) Europäisches Patentamt
European Patent Office
Office européen des brevets

(11) Veröffentlichungsnummer: **0 414 112 B1**

(12) **EUROPÄISCHE PATENTSCHRIFT**

(45) Veröffentlichungstag der Patentschrift: **20.12.95**

(51) Int. Cl.[6]: **C07D 513/04**, A01N 43/90, //(C07D513/04,285:00,237:00)

(21) Anmeldenummer: **90115625.7**

(22) Anmeldetag: **16.08.90**

(54) **Herbizide Thiadiaza-bicyclononan-Derivate**

(30) Priorität: **19.08.89 DE 3927388**

(43) Veröffentlichungstag der Anmeldung:
**27.02.91 Patentblatt 91/09**

(45) Bekanntmachung des Hinweises auf die Patenterteilung:
**20.12.95 Patentblatt 95/51**

(84) Benannte Vertragsstaaten:
**BE CH DE ES FR GB IT LI NL**

(56) Entgegenhaltungen:
**EP-A- 0 273 417**
**EP-A- 0 304 920**
**EP-A- 0 319 791**
**EP-A- 0 320 677**

(73) Patentinhaber: **BASF Aktiengesellschaft**
**Carl-Bosch-Strasse 38**
**D-67063 Ludwigshafen (DE)**

(72) Erfinder: **Rueb, Lothar, Dr.**
**Am Schoeneck 11**
**D-6720 Speyer (DE)**
Erfinder: **Eicken, Karl, Dr.**
**Am Huettenwingert 12**
**D-6706 Wachenheim (DE)**
Erfinder: **Westphalen, Karl-Otto, Dr.**
**Mausbergweg 58**
**D-6720 Speyer (DE)**
Erfinder: **Wuerzer, Bruno, Dr.**
**Ruedigerstrasse 13**
**D-6701 Otterstadt (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft neue Thiadiaza-bicyclononan-Derivate der allgemeinen Formel I

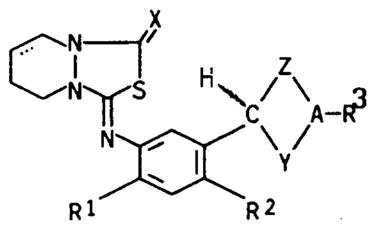

I

in der die punktierte Bindung für eine mögliche zusätzliche Bindung steht und die Substituenten folgende Bedeutung haben:

- $R^1$ Wasserstoff oder Fluor;
- $R^2$ Halogen;
- $R^3$ Wasserstoff, eine $C_1$-$C_8$-Alkoxycarbonylgruppe, eine $C_3$-$C_6$-Alkenyloxycarbonylgruppe oder eine $C_3$-$C_6$-Alkinyloxycarbonylgruppe, wobei diese 3 Gruppen jeweils noch einen $C_1$-$C_4$-Alkoxyrest tragen können;
- X,Y,Z Sauerstoff oder Schwefel;
- A eine $C_2$-$C_3$-Alkylenkette, welche zusätzlich zu $R^3$ bis zu drei gleiche oder verschiedene Reste $R^4$ tragen kann: Hydroxyl, Carboxyl, $C_1$-$C_4$-Alkyl oder $C_1$-$C_6$-Alkoxycarbonyl, wobei die beiden letzten genannten Reste ihrerseits bis zu 5 Halogenatome und einen der folgenden Substituenten tragen können: Hydroxyl, Cyan, Mercapto, $C_1$-$C_4$-Alkoxy, $C_1$-$C_6$-Alkylthio, $C_3$-$C_6$-Alkenyloxy, $C_3$-$C_6$-Alkenylthio, $C_3$-$C_6$-Alkinyloxy, $C_3$-$C_6$-Alkinylthio, $C_1$-$C_6$-Alkylcarbonyloxy, $C_1$-$C_6$-Alkoxycarbonyl, $C_1$-$C_6$-Alkoxycarbonyl-$C_1$-$C_4$-alkoxy oder $C_1$-$C_6$-Alkoxycarbonyl-$C_1$-$C_4$-allkylthio.

Des weiteren betrifft die Erfindung Verfahren zur Herstellung dieser Verbindungen sowie deren Verwendung als herbizide Mittel.

Herbizide Acetale und Thioacetale des Benzaldehyds, dessen Phenylring in meta-Stellung zur Acetalgruppe durch einen Tetrahydrophthalimid-Rest substituiert ist und der außerdem vergleichbare Substituenten $R^1$ und $R^2$ trägt, sind aus der EP-A-207 894, EP-A-03 19 791, DE-A-37 41 273 und der Deutschen Patentanmeldung DE-A- (P 3916292.3) bekannt.

Gemäß der Lehre der EP-A-0 273 417 sind neben bestimmten 9-(3-Oxo-2H-benzoxazinylimino)-8-thia-1,6-diazabicyclo[4.3.0]-nonan-7-on-Derivaten allgemein Verbindungen der Formel

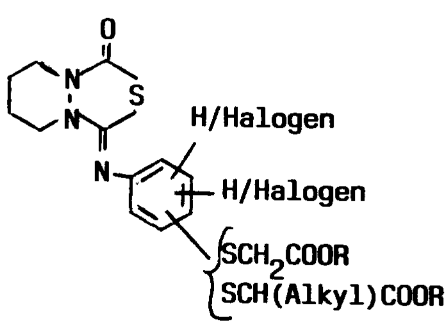

(R = Alkyl, Cycloalkyl, Alkoxyalkyl oder Alkoxycarbonyl) als Herbizide brauchbar.

In der EP-A-0 304 920 wird ferner beschrieben, daß 8-Thia-1,6-diazabicyclo[4.3.0]4-nonen-7-(thi)one, die in 9-Stellung eine 3-Oxo-2H-1,4-benzoxazin-6-ylimino)-, 3-Oxo-2H-1,4-benzthiazin-6-ylimino)- oder eine substituierte 4-Halogenphenylimino-Gruppe tragen, ebenfalls herbizid wirksam sind. Unter die allgemeine Definition dieser Verbindungen fallen u.a. Ketale und Thioketale der Formel

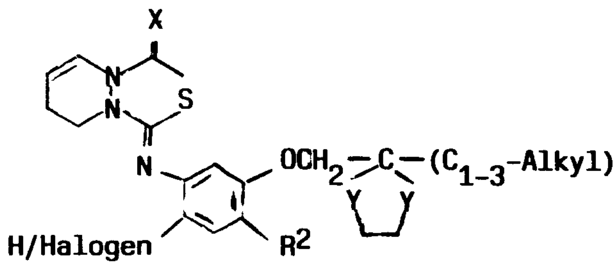

Es sind jedoch Verbindungen wünschenswert, die bei geringeren Aufwandmengen gute herbizide Wirkung zeigen.

Der Erfindung lagen daher neue Substanzen mit verbesserter herbizider Wirkung als Aufgabe zugrunde.

Demgemäß wurden die eingangs definierten Thiadiaza-bicyclononan-Derivate I gefunden.

Außerdem wurden Verfahren zur Herstellung der Verbindungen I, die Verwendung dieser Verbindungen als Herbizide sowie diese enthaltene herbizide Mittel gefunden.

Man erhält die Verbindungen I beispielsweise dadurch, daß man ein Nitrobenzol der allgemeinen Formel II in an sich bekannter Weise (Ferri, Reaktionen der org. Synthese; G. Thieme Verlag Stuttgart, S. 27ff.) in einem inerten organischen Lösungsmittel in Gegenwart einer Säure bei Temperaturen von 0 bis 180 °C, vorzugsweise 25 bis 150 °C mit einer Verbindung IIIa acetalisiert, das so erhaltene Acetal bzw. Thioacetal IV anschließend zum Anilinderivat V reduziert und V mit Thiophosgen zum Isothiocyanat VI umsetzt, schließlich ein cyclisches Hydrazin der Formel VII addiert und cyclisiert.

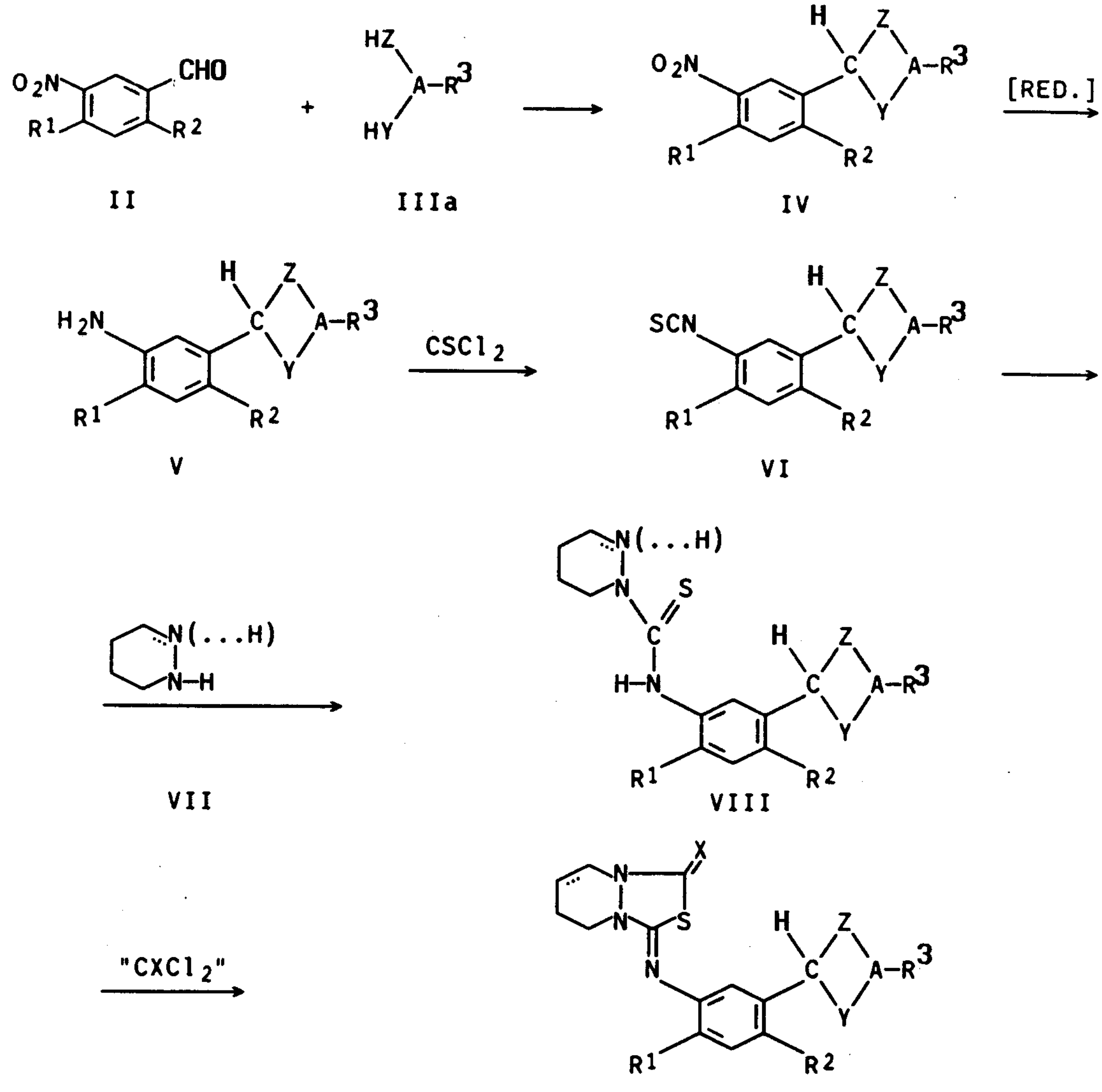


II IIIa IV V VI VII VIII I

Die Acetalisierung von II mit IIIa wird bevorzugt in einem aprotischen organischen Lösungsmittel wie Toluol und Xylol, insbesondere Toluol, in Gegenwart einer Säure wie p-Toluolsulfonsäure als Katalysator durchgeführt.

Die Reduktion des so erhaltenen Acetals IV zum Anilinderivat V wird nach allgemein üblichen Methoden (Houben-Weyl, Bd. 4/1c, S. 507ff. und Bd. 4/1d, S. 470ff), beispielsweise in Gegenwart von anorganischen Reduktionsmitteln wie Eisen und Zinn-II-salzen in aprotisch polaren Lösungsmitteln oder in Gegenwart von Wasserstoff an Metallkontakten wie Platin, Palladium und Raney-Nickel in aprotisch polaren Lösungsmitteln vorgenommen.

Die Darstellung der Isothiocyanate VI erfolgt in an sich bekannter Weise bei 0-100 °C, vorzugsweise bei 20-50 °C, in einem Zweiphasensystem wie zum Beispiel Methylenchlorid/Wasser oder in einem inerten Lösungsmittel wie Toluol unter Zusatz einer org. Base, z.B. tert. Aminen wie Triethylamin.

Die Isothiocyanate VI werden mit den Verbindungen der Formel VII in aprotisch polaren Lösungsmitteln wie Tetrahydrofuran, bei Temperaturen um 0 bis 100 °C, vorzugsweise bei 20-50 °C umgesetzt. Die Cyclisierung zu den Verbindungen der allgemeinen Formel I geschieht bei 0 bis 100 °C, vorzugsweise bei 20-70 °C in einem Lösungsmittel wie Methylenchlorid, unter Zusatz einer Base wie Pyridin, durch Zugabe eines Phosgenierungsmittels wie Phosgen, Thiophosgen oder Chlorameisensäuretrichlormethylester.

Im allgemeinen arbeitet man bei Atmosphärendruck oder unter dem Eigendruck des jeweiligen Lösungsmittels.

Bedeutet $R^3$ Wasserstoff und A eine Ethylen- oder Propylenkette, welche ein bis drei $C_1$-$C_3$-Alkylgruppen, vorzugsweise Methyl und Ethyl, tragen kann, so können diese Thiadiaza-bicyclononan-Derivate I in an sich bekannter Weise zu weiteren erfindungsgemäßen Verbindungen I umacetalisiert werden.

Im allgemeinen betragen die molaren Verhältnisse der Ausgangsverbindungen der einzelnen Stufen 1:1 bis 1:2.

Zur Reduktion der Nitroverbindung IV zum Anilinderivat V empfehlen sich äquimolare Mengen oder ein überschuß an Reduktionsmittel bis zu etwa 5facher Menge, bevorzugt 3facher Menge, bezogen auf die Menge an Verbindung IV.

Die Konzentration der Edukte im Lösungsmittel beträgt im allgemeinen 0,05 bis 5 mol/l, bevorzugt 0,1 bis 2,5 mol/l.

Die benötigten Edukte sind im allgemeinen bekannt oder nach bekannten Methoden herstellbar.

Im Hinblick auf die bestimmungsgemäße Verwendung der Verbindungen I als Herbizide kommen als Substituenten bevorzugt folgende Reste in Betracht:

$R^1$ Wasserstoff oder Fluor, vorzugsweise Wasserstoff;

$R^2$ Halogen wie Fluor, Chlor, Brom und Jod, vorzugsweise Fluor und Chlor;

$R^3$ Wasserstoff,

Alkoxycarbonyl wie Methoxycarbonyl, Ethoxycarbonyl, Propyloxycarbonyl, 1-Methylethoxycarbonyl, Butyloxycarbonyl, 1-Methylpropyloxycarbonyl, 2-Methylpropyloxycarbonyl, 1,1-Dimethylethoxycarbonyl, Pentoxycarbonyl, 1-Methylbutoxycarbonyl, 2-Methylbutoxycarbonyl, 3-Methylbutoxycarbonyl,1,2-Dimethylpropyloxycarbonyl, 1,1-Dimethylpropyloxycarbonyl, 2,2-Dimethylpropyloxycarbonyl, 1-Ethylpropyloxycarbonyl, n-Hexyloxycarbonyl, 1-Methyl-pentyloxycarbonyl, 2-Methylpentyloxycarbonyl, 3-Methylpentyloxycarbonyl, 4-Methylpentyloxycarbonyl, 1,2-Dimethylbutyloxycarbonyl, 1,3-Dimethylbutoxycarbonyl, 2,3-Dimethylbutoxycarbonyl, 1,1-Dimethylbutoxycarbonyl, 2,2-Dimethylbutoxycarbonyl, 3,3-Dimethylbutoxycarbonyl, 1,1,2-Trimethylpropyloxycarbonyl, 1,2,2-Trimethylpropyloxycarbonyl, 1-Ethylbutoxycarbonyl, 2-Ethylbutoxycarbonyl und 1-Ethyl-2-methylpropyloxycarbonyl;

Alkenyloxycarbonyl wie 2-Propenyloxycarbonyl, 2-Butenyloxycarbonyl, 3-Butenyloxycarbonyl, 1-Methyl-2-propenyloxycarbonyl, 2-Methyl-2-propenyloxycarbonyl, 2-Pentenyloxycarbonyl, 3-Pentenyloxycarbonyl, 4-Pentenyloxycarbonyl, 1-Methyl-2-butenyloxycarbonyl, 2-Methyl-2-butenyloxycarbonyl, 3-Methyl-2-butenyloxycarbonyl, 1-Methyl-3-butenyloxycarbonyl, 2-Methyl-3-butenyloxycarbonyl, 3-Methyl-3-butenyloxycarbonyl, 1,1-Dimethyl-2-propenyloxycarbonyl, 1,2-Dimethyl-2-propenyloxycarbonyl, 1-Ethyl-2-propenyloxycarbonyl, 2-Hexenyloxycarbonyl, 3-Hexenyloxycarbonyl, 4-Hexenyloxycarbonyl, 5-Hexenyloxycarbonyl, 1-Methyl-2-pentenyloxycarbonyl, 2-Methyl-2-pentenyloxycarbonyl, 3-Methyl-2-pentenyloxycarbonyl, 4-Methyl-2-pentenyloxycarbonyl, 1-Methyl-3-pentenyloxycarbonyl, 2-Methyl-3-pentenyloxycarbonyl, 3-Methyl-3-pentenyloxycarbonyl, 4-Methyl-3-pentenyloxycarbonyl, 1-Methyl-4-pentenyloxycarbonyl, 2-Methyl-4-pentenyloxycarbonyl, 3-Methyl-4-pentenyloxycarbonyl, 4-Methyl-4-pentenyloxycarbonyl, 1,1-Dimethyl-2-butenyloxycarbonyl, 1,1-Dimethyl-3-butenyloxycarbonyl, 1,2-Dimethyl-2-butenyloxycarbonyl, 1,2-Dimethyl-3-butenyloxycarbonyl, 1,3-Dimethyl-2-butenyloxycarbonyl, 1,3-Dimethyl-3-butenyloxycarbonyl, 2,2-Dimethyl-3-butenyloxycarbonyl, 2,3-Dimethyl-2-butenyloxycarbonyl, 2,3-Dimethyl-3-butenyloxycarbonyl, 1-Ethyl-2-butenyloxycarbonyl, 1-Ethyl-3-butenyloxycarbonyl, 2-Ethyl-2-butenyloxycarbonyl, 2-Ethyl-3-butenyloxycarbonyl, 1,1,2-Trimethyl-2-propenyloxycarbonyl, 1-Ethyl-1-methyl-2-propenyloxycarbonyl und 1-Ethyl-2-methyl-2-propenyloxycarbonyl;

Alkinyloxycarbonyl wie 2-Propinyloxycarbonyl, 2-Butinyloxycarbonyl, 3-Butinyloxycarbonyl, 1-Methyl-2-propinyloxycarbonyl, 2-Pentinyloxycarbonyl, 3-Pentinyloxycarbonyl, 4-Pentinyloxycarbonyl, 1-Methyl-3-butinyloxycarbonyl, 2-Methyl-3-butinyloxycarbonyl, 1-Methyl-2-butinyloxycarbonyl, 1,1-Dimethyl-2-propinyloxycarbonyl, 1-Ethyl-2-propinyloxycarbonyl, 2-Hexinyloxycarbonyl, 3-Hexinyloxycarbonyl, 4-Hexinyloxycarbonyl, 5-Hexinyloxycarbonyl, 1-Methyl-2-pentinyloxycarbonyl, 1-Methyl-3-pentinyloxycarbonyl, 1-Methyl-4-pentinyloxycarbonyl, 2-Methyl-3-pentinyloxycarbonyl, 2-Methyl-4-pentinyloxycarbonyl, 3-Methyl-4-pentinyloxycarbonyl, 4-Methyl-2-pentinyloxycarbonyl, 1,1-Dimethyl-2-butinyloxycarbonyl, 1,1-Dimethyl-3-butinyloxycarbonyl, 1,2-Dimethyl-3-butinyloxycarbonyl, 2,2-Dimethyl-3-butinyloxycarbonyl, 1-Ethyl-2-butinyloxycarbonyl, 1-Ethyl-3-butinyloxycarbonyl, 2-Ethyl-3-butinyloxycarbonyl und 1-Ethyl-1-methyl-2-propinyloxycarbonyl;

wobei diese Gruppen zusätzlich einen Alkoxyrest wie Methoxy, Ethoxy, Propyloxy, 1-Methylethoxy, Butyloxy, 1-Methylpropyloxy, 2-Methylpropyloxy und 1,1-Dimethylethoxy, vorzugsweise Methoxy und Ethoxy, tragen können;

X,Y Sauerstoff oder Schwefel, vorzugsweise Sauerstoff;

A Alkylen wie 1,2-Ethylen und 1,3-Propylen, welches ein bis drei der folgenden Reste tragen kann:

Hydroxy; Carboxyl;

Alkyl wie Methyl, Ethyl, n-Propyl, Isopropyl, n-Butyl, i-Butyl, sec.-Butyl und tert.-Butyl, vorzugsweise Methyl und Ethyl, oder Alkoxycarbonyl wie bei $R^3$ im allgemeinen und im besonderen

genannt, wobei die beiden letztgenannten Reste ihrerseits ein bis fünf Halogenatome wie bei $R^2$ genannt oder einen der folgenden Reste tragen können: Cyano; Mercapto; Alkoxy wie bei $R^3$ genannt; Alkenyloxy wie 2-Propenyloxy, 2-Butenyloxy, 3-Butenyloxy, 1-Methyl-2-propenyloxy, 2-Methyl-2-propenyloxy, 2-Pentenyloxy, 3-Pentenyloxy, 4-Pentenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-2-propenyloxy, 1-Ethyl-2-propenyloxy, 2-Hexenyloxy, 3-Hexenyloxy, 4-Hexenyloxy, 5-Hexenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,1-Dimethyl-3-butenyloxy, 1,2-Dimethyl-2-butenyloxy, 1,2-Dimethyl-3-butenyloxy, 1,3-Dimethyl-1-butenyloxy, 1,3-Dimethyl-2-butenyloxy, 1,3-Dimethyl-3-butenyloxy, 2,2-Dimethyl-3-butenyloxy, 2,3-Dimethyl-2-butenyloxy, 2,3-Dimethyl-3-butenyloxy, 1-Ethyl-2-butenyloxy, 1-Ethyl-3-butenyloxy, 2-Ethyl-2-butenyloxy, 2-Ethyl-3-butenyloxy, 1,1,2-Trimethyl-2-propenyloxy, 1-Ethyl-1-methyl-2-propenyloxy und 1-Ethyl-2-methyl-2-propenyloxy; Alkinyloxy wie 2-Propinyloxy, 2-Butinyloxy, 3-Butinyloxy, 1-Methyl-2-propinyloxy, 2-Pentinyloxy, 3-Pentinyloxy, 4-Pentinyloxy, 1-Methyl-3-butinyloxy, 2-Methyl-3-butinyloxy, 1-Methyl-2-butinyloxy, 1,1-Dimethyl-2-propinyloxy, 1-Ethyl-2-propinyloxy, 2-Hexinyloxy, 3-Hexinyloxy, 4-Hexinyloxy, 5-Hexinyloxy, 1-Methyl-2-pentinyloxy, 1-Methyl-3-pentinyloxy, 1-Methyl-4-pentinyloxy, 2-Methyl-3-pentinyloxy, 2-Methyl-4-pentinyloxy, 3-Methyl-4-pentinyloxy, 4-Methyl-2-pentinyloxy, 1,1-Dimethyl-2-butinyloxy, 1,1-Dimethyl-3-butinyloxy, 1,2-Dimethyl-3-butinyloxy, 2,2-Dimethyl-3-butinyloxy, 1-Ethyl-2-butinyloxy, 1-Ethyl-3-butinyloxy, 2-Ethyl-3-butinyloxy und 1-Ethyl-1-methyl-2-propinyloxy; Alkylcarbonyloxy wie Methylcarbonyloxy, Ethylcarbonyloxy, Propylcarbonyloxy, 1-Methylethylcarbonyloxy, Butylcarbonyloxy, 1-Methylpropylcarbonyloxy, 2-Methylpropylcarbonyloxy, 1,1-Dimethylethylcarbonyloxy, Pentylcarbonyloxy, 1-Methylbutylcarbonyloxy, 2-Methylbutylcarbonyloxy, 3-Methylbutylcarbonyloxy, 1,2-Dimethylpropylcarbonyloxy, 1,1-Dimethylpropylcarbonyloxy, 2,2-Dimethylpropylcarbonyloxy, 1-Ethylpropylcarbonyloxy, n-Hexylcarbonyloxy, 1-Methyl-pentylcarbonyloxy, 2-Methylpentylcarbonyloxy, 3-Methylpentylcarbonyloxy, 4-Methylpentylcarbonyloxy, 1,2-Dimethylbutylcarbonyloxy, 1,3-Dimethylbutylcarbonyloxy, 2,3-Dimethylbutylcarbonyloxy, 1,1-Dimethylbutylcarbonyloxy, 2,2-Dimethylbutylcarbonyloxy, 3,3-Dimethylbutylcarbonyloxy, 1,1,2-Trimethylpropylcarbonyloxy, 1,2,2-Trimethylpropylcarbonyloxy, 1-Ethylbutylcarbonyloxy, 2-Ethylbutylcarbonyloxy, und 1-Ethyl-2-methylpropylcarbonyloxy; Alkylthio wie Methylthio, Ethylthio, 1-Methylethylthio, Butylthio, 1-Methylpropylthio, 2-Methylpropylthio und 1,1-Dimethylethylthio; Alkenylthio wie 2-Propenylthio, 2-Butenylthio, 3-Butenylthio, 1-Methyl-2-propenylthio, 2-Methyl-2-propenylthio, 2-Pentenylthio, 3-Pentenylthio, 4-Pentenylthio, 1-Methyl-2-butenylthio, 2-Methyl-2-butenylthio, 3-Methyl-2-butenylthio, 1-Methyl-3-butenylthio, 2-Methyl-3-butenylthio, 3-Methyl-3-butenylthio, 1,1-Dimethyl-2-propenylthio, 1,2-Dimethyl-2-propenylthio, 1-Ethyl-2-propenylthio, 2-Hexenylthio, 3-Hexenylthio, 4-Hexenylthio, 5-Hexenylthio, 1-Methyl-2-pentenylthio, 2-Methyl-2-pentenylthio, 3-Methyl-2-pentenylthio, 4-Methyl-2-pentenylthio, 1-Methyl-3-pentenylthio, 2-Methyl-3-pentenylthio, 3-Methyl-3-pentenylthio, 4-Methyl-3-pentenylthio, 1-Methyl-4-pentenylthio, 2-Methyl-4-pentenylthio, 3-Methyl-4-pentenylthio, 4-Methyl-4-pentenylthio, 1,1-Dimethyl-2-butenylthio, 1,1-Dimethyl-3-butenylthio, 1,2-Dimethyl-2-butenylthio, 1,2-Dimethyl-3-butenylthio, 1,3-Dimethyl-1-butenylthio, 1,3-Dimethyl-2-butenylthio, 1,3-Dimethyl-3-butenylthio, 2,2-Dimethyl-3-butenylthio, 2,3-Dimethyl-2-butenylthio, 2,3-Dimethyl-3-butenylthio, 1-Ethyl-2-butenylthio, 1-Ethyl-3-butenylthio, 2-Ethyl-2-butenylthio, 2-Ethyl-3-butenylthio, 1,1,2-Trimethyl-2-propenylthio, 1-Ethyl-1-methyl-2-propenylthio und 1-Ethyl-2-methyl-2-propenylthio; Alkinylthio wie 2-Propinylthio, 2-Butinylthio, 3-Butinylthio, 1-Methyl-2-propinylthio, 2-Pentinylthio, 3-Pentinylthio, 4-Pentinylthio, 1-Methyl-3-butinylthio, 2-Methyl-3-butinylthio, 1-Methyl-2-butinylthio, 1,1-Dimethyl-2-propinylthio, 1-Ethyl-2-propinylthio, 2-Hexinylthio, 3-Hexinylthio, 4-Hexinylthio, 5-Hexinylthio, 1-Methyl-2-pentinylthio, 1-Methyl-3-pentinylthio, 1-Methyl-4-pentinylthio, 2-Methyl-3-pentinylthio, 2-Methyl-4-pentinylthio, 3-Methyl-4-pentinylthio, 4-Methyl-2-pentinylthio, 1,1-Dimethyl-2-butinylthio, 1,1-Dimethyl-3-butinylthio, 1,2-Dimethyl-3-butinylthio, 2,2-Dimethyl-3-butinylthio, 1-Ethyl-2-butinylthio, 1-Ethyl-3-butinylthio, 2-Ethyl-3-butinylthio und 1-Ethyl-1-methyl-2-propinylthio oder Alkoxycarbonyl wie bei $R^4$ genannt welches direkt oder über eine der vorstehend genannten Alkoxygruppen oder Alkylthiogruppen gebunden sein kann.

Besonders bevorzugt werden Verbindungen der Formeln Ia bis If (m = 0 oder 1).

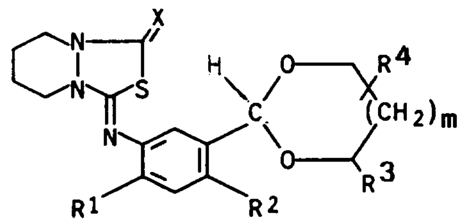

Ia

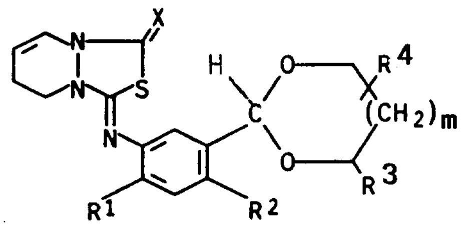

Ib

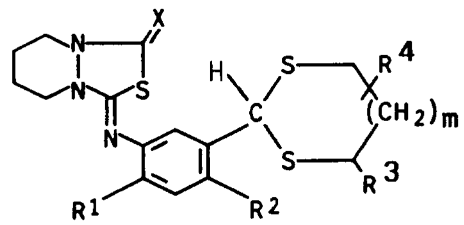

Ic

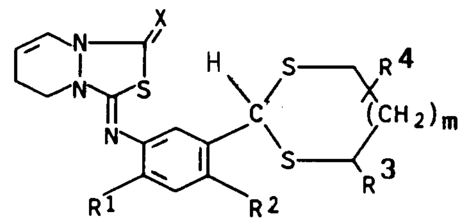

Id

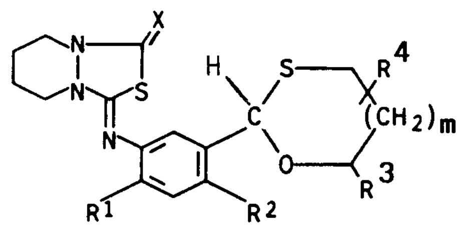

Ie

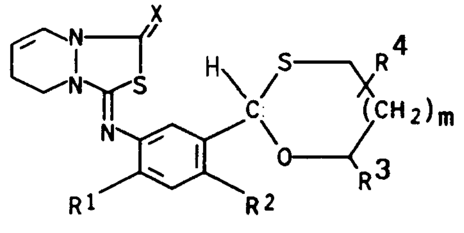

If

In Tabelle 1 sind beispielhaft mögliche Substituenten der besonders bevorzugten Verbindungen Ia bis Id aufgeführt, in Tabelle 2 mögliche Substituenten der besonders bevorzugten Verbindungen Ie und If.

Tabelle 1

| X | $R^1$ | $R^2$ | $R^3$ | m | $R^4$ |
|---|---|---|---|---|---|
| S | H | F | H | 0 | H |
| O | H | F | H | 0 | H |
| S | F | F | H | 0 | H |
| O | F | F | H | 0 | H |
| S | H | Cl | H | 0 | H |
| O | H | Cl | H | 0 | H |
| S | F | Cl | H | 0 | H |
| O | F | Cl | H | 0 | H |

| X | $R^1$ | $R^2$ | $R^3$ | m | $R^4$ |
|---|---|---|---|---|---|
| S | H | F | H | 0 | $4-CH_3$ |
| O | H | F | H | 0 | $4-CH_3$ |
| S | F | F | H | 0 | $4-CH_3$ |
| O | F | F | H | 0 | $4-CH_3$ |
| S | H | Cl | H | 0 | $4-CH_3$ |
| O | H | Cl | H | 0 | $4-CH_3$ |
| S | F | Cl | H | 0 | $4-CH_3$ |
| O | F | Cl | H | 0 | $4-CH_3$ |
| S | H | F | H | 0 | $4,5-Di-CH_3$ |
| O | H | F | H | 0 | $4,5-Di-CH_3$ |
| S | F | F | H | 0 | $4,5-Di-CH_3$ |
| O | F | F | H | 0 | $4,5-Di-CH_3$ |
| S | H | Cl | H | 0 | $4,5-Di-CH_3$ |
| O | H | Cl | H | 0 | $4,5-Di-CH_3$ |
| S | F | Cl | H | 0 | $4,5-Di-CH_3$ |
| O | F | Cl | H | 0 | $4,5-Di-CH_3$ |

| X | $R^1$ | $R^2$ | $R^3$ | m | $R^4$ |
|---|---|---|---|---|---|
| S | H | F | H | 0 | 4-$CH_2CH_3$ |
| O | H | F | H | 0 | 4-$CH_2CH_3$ |
| S | F | F | H | 0 | 4-$CH_2CH_3$ |
| O | F | F | H | 0 | 4-$CH_2CH_3$ |
| S | H | Cl | H | 0 | 4-$CH_2CH_3$ |
| O | H | Cl | H | 0 | 4-$CH_2CH_3$ |
| S | F | Cl | H | 0 | 4-$CH_2CH_3$ |
| O | F | Cl | H | 0 | 4-$CH_2CH_3$ |
| S | H | F | H | 0 | 4-$CH_2Cl$ |
| O | H | F | H | 0 | 4-$CH_2Cl$ |
| S | F | F | H | 0 | 4-$CH_2Cl$ |
| O | F | F | H | 0 | 4-$CH_2Cl$ |
| S | H | Cl | H | 0 | 4-$CH_2Cl$ |
| O | H | Cl | H | 0 | 4-$CH_2Cl$ |
| S | F | Cl | H | 0 | 4-$CH_2Cl$ |
| O | F | Cl | H | 0 | 4-$CH_2Cl$ |

| X | $R^1$ | $R^2$ | $R^3$ | m | $R^4$ |
|---|---|---|---|---|---|
| S | H | F | H | 0 | 4-$CH_2OH$ |
| O | H | F | H | 0 | 4-$CH_2OH$ |
| S | F | F | H | 0 | 4-$CH_2OH$ |
| O | F | F | H | 0 | 4-$CH_2OH$ |
| S | H | Cl | H | 0 | 4-$CH_2OH$ |
| O | H | Cl | H | 0 | 4-$CH_2OH$ |
| S | F | Cl | H | 0 | 4-$CH_2OH$ |
| O | F | Cl | H | 0 | 4-$CH_2OH$ |
| S | H | F | H | 0 | 4-$CH_2OCH_3$ |
| O | H | F | H | 0 | 4-$CH_2OCH_3$ |
| S | F | F | H | 0 | 4-$CH_2OCH_3$ |
| O | F | F | H | 0 | 4-$CH_2OCH_3$ |
| S | H | Cl | H | 0 | 4-$CH_2OCH_3$ |
| O | H | Cl | H | 0 | 4-$CH_2OCH_3$ |
| S | F | Cl | H | 0 | 4-$CH_2OCH_3$ |
| O | F | Cl | H | 0 | 4-$CH_2OCH_3$ |
| S | H | F | H | 0 | 4-$CH_2OCH_2CH=CH_2$ |
| O | H | F | H | 0 | 4-$CH_2OCH_2CH=CH_2$ |

| X | $R^1$ | $R^2$ | $R^3$ | m | $R^4$ |
|---|---|---|---|---|---|
| S | F | F | H | 0 | $4-CH_2OCH_2CH=CH_2$ |
| O | F | F | H | 0 | $4-CH_2OCH_2CH=CH_2$ |
| S | H | Cl | H | 0 | $4-CH_2OCH_2CH=CH_2$ |
| O | H | Cl | H | 0 | $4-CH_2OCH_2CH=CH_2$ |
| S | F | Cl | H | 0 | $4-CH_2OCH_2CH=CH_2$ |
| O | F | Cl | H | 0 | $4-CH_2OCH_2CH=CH_2$ |
| S | H | F | H | 0 | $4-CH_2OCH_2C\equiv CH$ |
| O | H | F | H | 0 | $4-CH_2OCH_2C\equiv CH$ |
| S | F | F | H | 0 | $4-CH_2OCH_2C\equiv CH$ |
| O | F | F | H | 0 | $4-CH_2OCH_2C\equiv CH$ |
| S | H | Cl | H | 0 | $4-CH_2OCH_2C\equiv CH$ |
| O | H | Cl | H | 0 | $4-CH_2OCH_2C\equiv CH$ |
| S | F | Cl | H | 0 | $4-CH_2OCH_2C\equiv CH$ |
| O | F | Cl | H | 0 | $4-CH_2OCH_2C\equiv CH$ |
| S | H | F | H | 0 | $4-CH_2OCOCH_3$ |
| O | H | F | H | 0 | $4-CH_2OCOCH_3$ |
| S | F | F | H | 0 | $4-CH_2OCOCH_3$ |
| O | F | F | H | 0 | $4-CH_2OCOCH_3$ |

| X | $R^1$ | $R^2$ | $R^3$ | m | $R^4$ |
|---|---|---|---|---|---|
| S | H | Cl | H | 0 | $4-CH_2OCOCH_3$ |
| O | H | Cl | H | 0 | $4-CH_2OCOCH_3$ |
| S | F | Cl | H | 0 | $4-CH_2OCOCH_3$ |
| O | F | Cl | H | 0 | $4-CH_2OCOCH_3$ |
| S | H | F | H | 0 | $4-CH_2OCH_2CO_2CH_3$ |
| O | H | F | H | 0 | $4-CH_2OCH_2CO_2CH_3$ |
| S | F | F | H | 0 | $4-CH_2OCH_2CO_2CH_3$ |
| O | F | F | H | 0 | $4-CH_2OCH_2CO_2CH_3$ |
| S | H | Cl | H | 0 | $4-CH_2OCH_2CO_2CH_3$ |
| O | H | Cl | H | 0 | $4-CH_2OCH_2CO_2CH_3$ |
| S | F | Cl | H | 0 | $4-CH_2OCH_2CO_2CH_3$ |
| O | F | Cl | H | 0 | $4-CH_2OCH_2CO_2CH_3$ |
| S | H | F | H | 0 | $4-CH_2CO_2CH_3$ |
| O | H | F | H | 0 | $4-CH_2CO_2CH_3$ |
| S | F | F | H | 0 | $4-CH_2CO_2CH_3$ |
| O | F | F | H | 0 | $4-CH_2CO_2CH_3$ |
| S | H | Cl | H | 0 | $4-CH_2CO_2CH_3$ |
| O | H | Cl | H | 0 | $4-CH_2CO_2CH_3$ |

| X | $R^1$ | $R^2$ | $R^3$ | m | $R^4$ |
|---|---|---|---|---|---|
| S | F | Cl | H | 0 | $4-CH_2CO_2CH_3$ |
| O | F | Cl | H | 0 | $4-CH_2CO_2CH_3$ |
| S | H | F | H | 0 | $4-CH_2SH$ |
| O | H | F | H | 0 | $4-CH_2SH$ |
| S | F | F | H | 0 | $4-CH_2SH$ |
| O | F | F | H | 0 | $4-CH_2SH$ |
| S | H | Cl | H | 0 | $4-CH_2SH$ |
| O | H | Cl | H | 0 | $4-CH_2SH$ |
| S | F | Cl | H | 0 | $4-CH_2SH$ |
| O | F | Cl | H | 0 | $4-CH_2SH$ |
| S | H | F | H | 0 | $4-CH_2SCH_2CH_3$ |
| O | H | F | H | 0 | $4-CH_2SCH_2CH_3$ |
| S | F | F | H | 0 | $4-CH_2SCH_2CH_3$ |
| O | F | F | H | 0 | $4-CH_2SCH_2CH_3$ |
| S | H | Cl | H | 0 | $4-CH_2SCH_2CH_3$ |
| O | H | Cl | H | 0 | $4-CH_2SCH_2CH_3$ |
| S | F | Cl | H | 0 | $4-CH_2SCH_2CH_3$ |
| O | F | Cl | H | 0 | $4-CH_2SCH_2CH_3$ |

| X | $R^1$ | $R^2$ | $R^3$ | m | $R^4$ |
|---|---|---|---|---|---|
| S | H | F | H | 0 | $4-CH_2SCH_2CH=CH_2$ |
| O | H | F | H | 0 | $4-CH_2SCH_2CH=CH_2$ |
| S | F | F | H | 0 | $4-CH_2SCH_2CH=CH_2$ |
| O | F | F | H | 0 | $4-CH_2SCH_2CH=CH_2$ |
| S | H | Cl | H | 0 | $4-CH_2SCH_2CH=CH_2$ |
| O | H | Cl | H | 0 | $4-CH_2SCH_2CH=CH_2$ |
| S | F | Cl | H | 0 | $4-CH_2SCH_2CH=CH_2$ |
| O | F | Cl | H | 0 | $4-CH_2SCH_2CH=CH_2$ |
| S | H | F | H | 0 | $4-CO_2H$ |
| O | H | F | H | 0 | $4-CO_2H$ |
| S | F | F | H | 0 | $4-CO_2H$ |
| O | F | F | H | 0 | $4-CO_2H$ |
| S | H | Cl | H | 0 | $4-CO_2H$ |
| O | H | Cl | H | 0 | $4-CO_2H$ |
| S | F | Cl | H | 0 | $4-CO_2H$ |
| O | F | Cl | H | 0 | $4-CO_2H$ |

| X | $R^1$ | $R^2$ | $R^3$ | m | $R^4$ |
|---|---|---|---|---|---|
| S | H | F | H | 1 | H |
| O | H | F | H | 1 | H |
| S | F | F | H | 1 | H |
| O | F | F | H | 1 | H |
| S | H | Cl | H | 1 | H |
| O | H | Cl | H | 1 | H |
| S | F | Cl | H | 1 | H |
| O | F | Cl | H | 1 | H |
| S | H | F | H | 1 | $4-CH_3$ |
| O | H | F | H | 1 | $4-CH_3$ |
| S | F | F | H | 1 | $4-CH_3$ |
| O | F | F | H | 1 | $4-CH_3$ |
| S | H | Cl | H | 1 | $4-CH_3$ |
| O | H | Cl | H | 1 | $4-CH_3$ |
| S | F | Cl | H | 1 | $4-CH_3$ |
| O | F | Cl | H | 1 | $4-CH_3$ |

| X | $R^1$ | $R^2$ | $R^3$ | m | $R^4$ |
|---|---|---|---|---|---|
| S | H | F | H | 1 | 4,6-Di-$CH_3$ |
| O | H | F | H | 1 | 4,6-Di-$CH_3$ |
| S | F | F | H | 1 | 4,6-Di-$CH_3$ |
| O | F | F | H | 1 | 4,6-Di-$CH_3$ |
| S | H | Cl | H | 1 | 4,6-Di-$CH_3$ |
| O | H | Cl | H | 1 | 4,6-Di-$CH_3$ |
| S | F | Cl | H | 1 | 4,6-Di-$CH_3$ |
| O | F | Cl | H | 1 | 4,6-Di-$CH_3$ |
| S | H | F | H | 1 | 4-$CH_2CH_3$ |
| O | H | F | H | 1 | 4-$CH_2CH_3$ |
| S | F | F | H | 1 | 4-$CH_2CH_3$ |
| O | F | F | H | 1 | 4-$CH_2CH_3$ |
| S | H | Cl | H | 1 | 4-$CH_2CH_3$ |
| O | H | Cl | H | 1 | 4-$CH_2CH_3$ |
| S | F | Cl | H | 1 | 4-$CH_2CH_3$ |
| O | F | Cl | H | 1 | 4-$CH_2CH_3$ |

| X | $R^1$ | $R^2$ | $R^3$ | m | $R^4$ |
|---|---|---|---|---|---|
| S | H | F | H | 1 | 4-$CH_2Cl$ |
| O | H | F | H | 1 | 4-$CH_2Cl$ |
| S | F | F | H | 1 | 4-$CH_2Cl$ |
| O | F | F | H | 1 | 4-$CH_2Cl$ |
| S | H | Cl | H | 1 | 4-$CH_2Cl$ |
| O | H | Cl | H | 1 | 4-$CH_2Cl$ |
| S | F | Cl | H | 1 | 4-$CH_2Cl$ |
| O | F | Cl | H | 1 | 4-$CH_2Cl$ |
| S | H | F | H | 1 | 4-$CH_2OH$ |
| O | H | F | H | 1 | 4-$CH_2OH$ |
| S | F | F | H | 1 | 4-$CH_2OH$ |
| O | F | F | H | 1 | 4-$CH_2OH$ |
| S | H | Cl | H | 1 | 4-$CH_2OH$ |
| O | H | Cl | H | 1 | 4-$CH_2OH$ |
| S | F | Cl | H | 1 | 4-$CH_2OH$ |
| O | F | Cl | H | 1 | 4-$CH_2OH$ |

| X | $R^1$ | $R^2$ | $R^3$ | m | $R^4$ |
|---|---|---|---|---|---|
| S | H | F | H | 1 | 4-$CH_2OCH_3$ |
| O | H | F | H | 1 | 4-$CH_2OCH_3$ |
| S | F | F | H | 1 | 4-$CH_2OCH_3$ |
| O | F | F | H | 1 | 4-$CH_2OCH_3$ |
| S | H | Cl | H | 1 | 4-$CH_2OCH_3$ |
| O | H | Cl | H | 1 | 4-$CH_2OCH_3$ |
| S | F | Cl | H | 1 | 4-$CH_2OCH_3$ |
| O | F | Cl | H | 1 | 4-$CH_2OCH_3$ |
| S | H | F | H | 1 | 4-$CH_2OCH_2CH=CH_2$ |
| O | H | F | H | 1 | 4-$CH_2OCH_2CH=CH_2$ |
| S | F | F | H | 1 | 4-$CH_2OCH_2CH=CH_2$ |
| O | F | F | H | 1 | 4-$CH_2OCH_2CH=CH_2$ |
| S | H | Cl | H | 1 | 4-$CH_2OCH_2CH=CH_2$ |
| O | H | Cl | H | 1 | 4-$CH_2OCH_2CH=CH_2$ |
| S | F | Cl | H | 1 | 4-$CH_2OCH_2CH=CH_2$ |
| O | F | Cl | H | 1 | 4-$CH_2OCH_2CH=CH_2$ |

| X | $R^1$ | $R^2$ | $R^3$ | m | $R^4$ |
|---|---|---|---|---|---|
| S | H | F | H | 1 | 4-$CH_2OCH_2C{\equiv}CH$ |
| O | H | F | H | 1 | 4-$CH_2OCH_2C{\equiv}CH$ |
| S | F | F | H | 1 | 4-$CH_2OCH_2C{\equiv}CH$ |
| O | F | F | H | 1 | 4-$CH_2OCH_2C{\equiv}CH$ |
| S | H | Cl | H | 1 | 4-$CH_2OCH_2C{\equiv}CH$ |
| O | H | Cl | H | 1 | 4-$CH_2OCH_2C{\equiv}CH$ |
| S | F | Cl | H | 1 | 4-$CH_2OCH_2C{\equiv}CH$ |
| O | F | Cl | H | 1 | 4-$CH_2OCH_2C{\equiv}CH$ |
| S | H | F | H | 1 | 4-$CH_2OCOCH_3$ |
| O | H | F | H | 1 | 4-$CH_2OCOCH_3$ |
| S | F | F | H | 1 | 4-$CH_2OCOCH_3$ |
| O | F | F | H | 1 | 4-$CH_2OCOCH_3$ |
| S | H | Cl | H | 1 | 4-$CH_2OCOCH_3$ |
| O | H | Cl | H | 1 | 4-$CH_2OCOCH_3$ |
| S | F | Cl | H | 1 | 4-$CH_2OCOCH_3$ |
| O | F | Cl | H | 1 | 4-$CH_2OCOCH_3$ |

| X | $R^1$ | $R^2$ | $R^3$ | m | $R^4$ |
|---|---|---|---|---|---|
| S | H | F | H | 1 | 4-$CH_2OCH_2CO_2CH_3$ |
| O | H | F | H | 1 | 4-$CH_2OCH_2CO_2CH_3$ |
| S | F | F | H | 1 | 4-$CH_2OCH_2CO_2CH_3$ |
| O | F | F | H | 1 | 4-$CH_2OCH_2CO_2CH_3$ |
| S | H | Cl | H | 1 | 4-$CH_2OCH_2CO_2CH_3$ |
| O | H | Cl | H | 1 | 4-$CH_2OCH_2CO_2CH_3$ |
| S | F | Cl | H | 1 | 4-$CH_2OCH_2CO_2CH_3$ |
| O | F | Cl | H | 1 | 4-$CH_2OCH_2CO_2CH_3$ |
| S | H | F | H | 1 | 4-$CH_2CO_2CH_3$ |
| O | H | F | H | 1 | 4-$CH_2CO_2CH_3$ |
| S | F | F | H | 1 | 4-$CH_2CO_2CH_3$ |
| O | F | F | H | 1 | 4-$CH_2CO_2CH_3$ |
| S | H | Cl | H | 1 | 4-$CH_2CO_2CH_3$ |
| O | H | Cl | H | 1 | 4-$CH_2CO_2CH_3$ |
| S | F | Cl | H | 1 | 4-$CH_2CO_2CH_3$ |
| O | F | Cl | H | 1 | 4-$CH_2CO_2CH_3$ |

| X | $R^1$ | $R^2$ | $R^3$ | m | $R^4$ |
|---|---|---|---|---|---|
| S | H | F | H | 1 | 4-$CH_2SH$ |
| O | H | F | H | 1 | 4-$CH_2SH$ |
| S | F | F | H | 1 | 4-$CH_2SH$ |
| O | F | F | H | 1 | 4-$CH_2SH$ |
| S | H | Cl | H | 1 | 4-$CH_2SH$ |
| O | H | Cl | H | 1 | 4-$CH_2SH$ |
| S | F | Cl | H | 1 | 4-$CH_2SH$ |
| O | F | Cl | H | 1 | 4-$CH_2SH$ |
| S | H | F | H | 1 | 4-$CH_2SCH_2CH_3$ |
| O | H | F | H | 1 | 4-$CH_2SCH_2CH_3$ |
| S | F | F | H | 1 | 4-$CH_2SCH_2CH_3$ |
| O | F | F | H | 1 | 4-$CH_2SCH_2CH_3$ |
| S | H | Cl | H | 1 | 4-$CH_2SCH_2CH_3$ |
| O | H | Cl | H | 1 | 4-$CH_2SCH_2CH_3$ |
| S | F | Cl | H | 1 | 4-$CH_2SCH_2CH_3$ |
| O | F | Cl | H | 1 | 4-$CH_2SCH_2CH_3$ |

| X | $R^1$ | $R^2$ | $R^3$ | m | $R^4$ |
|---|---|---|---|---|---|
| S | H | F | H | 1 | $4-CH_2SCH_2CH=CH_2$ |
| O | H | F | H | 1 | $4-CH_2SCH_2CH=CH_2$ |
| S | F | F | H | 1 | $4-CH_2SCH_2CH=CH_2$ |
| O | F | F | H | 1 | $4-CH_2SCH_2CH=CH_2$ |
| S | H | Cl | H | 1 | $4-CH_2SCH_2CH=CH_2$ |
| O | H | Cl | H | 1 | $4-CH_2SCH_2CH=CH_2$ |
| S | F | Cl | H | 1 | $4-CH_2SCH_2CH=CH_2$ |
| O | F | Cl | H | 1 | $4-CH_2SCH_2CH=CH_2$ |
| S | H | F | H | 1 | $4-CO_2H$ |
| O | H | F | H | 1 | $4-CO_2H$ |
| S | F | F | H | 1 | $4-CO_2H$ |
| O | F | F | H | 1 | $4-CO_2H$ |
| S | H | Cl | H | 1 | $4-CO_2H$ |
| O | H | Cl | H | 1 | $4-CO_2H$ |
| S | F | Cl | H | 1 | $4-CO_2H$ |
| O | F | Cl | H | 1 | $4-CO_2H$ |

| X | $R^1$ | $R^2$ | $R^3$ | m | $R^4$ |
|---|---|---|---|---|---|
| S | H | F | $CO_2CH_3$ | 0 | H |
| O | H | F | $CO_2CH_3$ | 0 | H |
| S | F | F | $CO_2CH_3$ | 0 | H |
| O | F | F | $CO_2CH_3$ | 0 | H |
| S | H | Cl | $CO_2CH_3$ | 0 | H |
| O | H | Cl | $CO_2CH_3$ | 0 | H |
| S | F | Cl | $CO_2CH_3$ | 0 | H |
| O | F | Cl | $CO_2CH_3$ | 0 | H |
| S | H | F | $CO_2CH_2CH_3$ | 0 | H |
| O | H | F | $CO_2CH_2CH_3$ | 0 | H |
| S | F | F | $CO_2CH_2CH_3$ | 0 | H |
| O | F | F | $CO_2CH_2CH_3$ | 0 | H |
| S | H | Cl | $CO_2CH_2CH_3$ | 0 | H |
| O | H | Cl | $CO_2CH_2CH_3$ | 0 | H |
| S | F | Cl | $CO_2CH_2CH_3$ | 0 | H |
| O | F | Cl | $CO_2CH_2CH_3$ | 0 | H |

| X | $R^1$ | $R^2$ | $R^3$ | m | $R^4$ |
|---|---|---|---|---|---|
| S | H | F | $CO_2(CH_2)_2CH_3$ | 0 | H |
| O | H | F | $CO_2(CH_2)_2CH_3$ | 0 | H |
| S | F | F | $CO_2(CH_2)_2CH_3$ | 0 | H |
| O | F | F | $CO_2(CH_2)_2CH_3$ | 0 | H |
| S | H | Cl | $CO_2(CH_2)_2CH_3$ | 0 | H |
| O | H | Cl | $CO_2(CH_2)_2CH_3$ | 0 | H |
| S | F | Cl | $CO_2(CH_2)_2CH_3$ | 0 | H |
| O | F | Cl | $CO_2(CH_2)_2CH_3$ | 0 | H |
| S | H | F | $CO_2CH(CH_3)_2$ | 0 | H |
| O | H | F | $CO_2CH(CH_3)_2$ | 0 | H |
| S | F | F | $CO_2CH(CH_3)_2$ | 0 | H |
| O | F | F | $CO_2CH(CH_3)_2$ | 0 | H |
| S | H | Cl | $CO_2CH(CH_3)_2$ | 0 | H |
| O | H | Cl | $CO_2CH(CH_3)_2$ | 0 | H |
| S | F | Cl | $CO_2CH(CH_3)_2$ | 0 | H |
| O | F | Cl | $CO_2CH(CH_3)_2$ | 0 | H |

| X | $R^1$ | $R^2$ | $R^3$ | m | $R^4$ |
|---|---|---|---|---|---|
| S | H | F | $CO_2(CH_2)_3CH_3$ | 0 | H |
| O | H | F | $CO_2(CH_2)_3CH_3$ | 0 | H |
| S | F | F | $CO_2(CH_2)_3CH_3$ | 0 | H |
| O | F | F | $CO_2(CH_2)_3CH_3$ | 0 | H |
| S | H | Cl | $CO_2(CH_2)_3CH_3$ | 0 | H |
| O | H | Cl | $CO_2(CH_2)_3CH_3$ | 0 | H |
| S | F | Cl | $CO_2(CH_2)_3CH_3$ | 0 | H |
| O | F | Cl | $CO_2(CH_2)_3CH_3$ | 0 | H |
| S | H | F | $CO_2(CH_2)_2OCH_3$ | 0 | H |
| O | H | F | $CO_2(CH_2)_2OCH_3$ | 0 | H |
| S | F | F | $CO_2(CH_2)_2OCH_3$ | 0 | H |
| O | F | F | $CO_2(CH_2)_2OCH_3$ | 0 | H |
| S | H | Cl | $CO_2(CH_2)_2OCH_3$ | 0 | H |
| O | H | Cl | $CO_2(CH_2)_2OCH_3$ | 0 | H |
| S | F | Cl | $CO_2(CH_2)_2OCH_3$ | 0 | H |
| O | F | Cl | $CO_2(CH_2)_2OCH_3$ | 0 | H |

| X | $R^1$ | $R^2$ | $R^3$ | m | $R^4$ |
|---|---|---|---|---|---|
| S | H | F | $CO_2(CH_2)_2OCH_2CH_3$ | 0 | H |
| O | H | F | $CO_2(CH_2)_2OCH_2CH_3$ | 0 | H |
| S | F | F | $CO_2(CH_2)_2OCH_2CH_3$ | 0 | H |
| O | F | F | $CO_2(CH_2)_2OCH_2CH_3$ | 0 | H |
| S | H | Cl | $CO_2(CH_2)_2OCH_2CH_3$ | 0 | H |
| O | H | Cl | $CO_2(CH_2)_2OCH_2CH_3$ | 0 | H |
| S | F | Cl | $CO_2(CH_2)_2OCH_2CH_3$ | 0 | H |
| O | F | Cl | $CO_2(CH_2)_2OCH_2CH_3$ | 0 | H |
| S | H | F | $CO_2CH_3$ | 0 | 4-$CH_3$ |
| O | H | F | $CO_2CH_3$ | 0 | 4-$CH_3$ |
| S | F | F | $CO_2CH_3$ | 0 | 4-$CH_3$ |
| O | F | F | $CO_2CH_3$ | 0 | 4-$CH_3$ |
| S | H | Cl | $CO_2CH_3$ | 0 | 4-$CH_3$ |
| O | H | Cl | $CO_2CH_3$ | 0 | 4-$CH_3$ |
| S | F | Cl | $CO_2CH_3$ | 0 | 4-$CH_3$ |
| O | F | Cl | $CO_2CH_3$ | 0 | 4-$CH_3$ |

| X | $R^1$ | $R^2$ | $R^3$ | m | $R^4$ |
|---|---|---|---|---|---|
| S | H | F | $CO_2CH_2CH_3$ | 0 | 4-$CH_3$ |
| O | H | F | $CO_2CH_2CH_3$ | 0 | 4-$CH_3$ |
| S | F | F | $CO_2CH_2CH_3$ | 0 | 4-$CH_3$ |
| O | F | F | $CO_2CH_2CH_3$ | 0 | 4-$CH_3$ |
| S | H | Cl | $CO_2CH_2CH_3$ | 0 | 4-$CH_3$ |
| O | H | Cl | $CO_2CH_2CH_3$ | 0 | 4-$CH_3$ |
| S | F | Cl | $CO_2CH_2CH_3$ | 0 | 4-$CH_3$ |
| O | F | Cl | $CO_2CH_2CH_3$ | 0 | 4-$CH_3$ |
| S | H | F | $CO_2(CH_2)_2CH_3$ | 0 | 4-$CH_3$ |
| O | H | F | $CO_2(CH_2)_2CH_3$ | 0 | 4-$CH_3$ |
| S | F | F | $CO_2(CH_2)_2CH_3$ | 0 | 4-$CH_3$ |
| O | F | F | $CO_2(CH_2)_2CH_3$ | 0 | 4-$CH_3$ |
| S | H | Cl | $CO_2(CH_2)_2CH_3$ | 0 | 4-$CH_3$ |
| O | H | Cl | $CO_2(CH_2)_2CH_3$ | 0 | 4-$CH_3$ |
| S | F | Cl | $CO_2(CH_2)_2CH_3$ | 0 | 4-$CH_3$ |
| O | F | Cl | $CO_2(CH_2)_2CH_3$ | 0 | 4-$CH_3$ |

| X | $R^1$ | $R^2$ | $R^3$ | m | $R^4$ |
|---|---|---|---|---|---|
| S | H | F | $CO_2CH(CH_3)_2$ | 0 | 4-$CH_3$ |
| O | H | F | $CO_2CH(CH_3)_2$ | 0 | 4-$CH_3$ |
| S | F | F | $CO_2CH(CH_3)_2$ | 0 | 4-$CH_3$ |
| O | F | F | $CO_2CH(CH_3)_2$ | 0 | 4-$CH_3$ |
| S | H | Cl | $CO_2CH(CH_3)_2$ | 0 | 4-$CH_3$ |
| O | H | Cl | $CO_2CH(CH_3)_2$ | 0 | 4-$CH_3$ |
| S | F | Cl | $CO_2CH(CH_3)_2$ | 0 | 4-$CH_3$ |
| O | F | Cl | $CO_2CH(CH_3)_2$ | 0 | 4-$CH_3$ |
| S | H | F | $CO_2(CH_2)_3CH_3$ | 0 | 4-$CH_3$ |
| O | H | F | $CO_2(CH_2)_3CH_3$ | 0 | 4-$CH_3$ |
| S | F | F | $CO_2(CH_2)_3CH_3$ | 0 | 4-$CH_3$ |
| O | F | F | $CO_2(CH_2)_3CH_3$ | 0 | 4-$CH_3$ |
| S | H | Cl | $CO_2(CH_2)_3CH_3$ | 0 | 4-$CH_3$ |
| O | H | Cl | $CO_2(CH_2)_3CH_3$ | 0 | 4-$CH_3$ |
| S | F | Cl | $CO_2(CH_2)_3CH_3$ | 0 | 4-$CH_3$ |
| O | F | Cl | $CO_2(CH_2)_3CH_3$ | 0 | 4-$CH_3$ |

| X | $R^1$ | $R^2$ | $R^3$ | m | $R^4$ |
|---|---|---|---|---|---|
| S | H | F | $CO_2(CH_2)_2OCH_3$ | 0 | 4-$CH_3$ |
| O | H | F | $CO_2(CH_2)_2OCH_3$ | 0 | 4-$CH_3$ |
| S | F | F | $CO_2(CH_2)_2OCH_3$ | 0 | 4-$CH_3$ |
| O | F | F | $CO_2(CH_2)_2OCH_3$ | 0 | 4-$CH_3$ |
| S | H | Cl | $CO_2(CH_2)_2OCH_3$ | 0 | 4-$CH_3$ |
| O | H | Cl | $CO_2(CH_2)_2OCH_3$ | 0 | 4-$CH_3$ |
| S | F | Cl | $CO_2(CH_2)_2OCH_3$ | 0 | 4-$CH_3$ |
| O | F | Cl | $CO_2(CH_2)_2OCH_3$ | 0 | 4-$CH_3$ |
| S | H | F | $CO_2(CH_2)_2OCH_2CH_3$ | 0 | 4-$CH_3$ |
| O | H | F | $CO_2(CH_2)_2OCH_2CH_3$ | 0 | 4-$CH_3$ |
| S | F | F | $CO_2(CH_2)_2OCH_2CH_3$ | 0 | 4-$CH_3$ |
| O | F | F | $CO_2(CH_2)_2OCH_2CH_3$ | 0 | 4-$CH_3$ |
| S | H | Cl | $CO_2(CH_2)_2OCH_2CH_3$ | 0 | 4-$CH_3$ |
| O | H | Cl | $CO_2(CH_2)_2OCH_2CH_3$ | 0 | 4-$CH_3$ |
| S | F | Cl | $CO_2(CH_2)_2OCH_2CH_3$ | 0 | 4-$CH_3$ |
| O | F | Cl | $CO_2(CH_2)_2OCH_2CH_3$ | 0 | 4-$CH_3$ |

| X | $R^1$ | $R^2$ | $R^3$ | m | $R^4$ |
|---|---|---|---|---|---|
| S | H | F | $CO_2CH_3$ | 0 | 5-$CH_3$ |
| O | H | F | $CO_2CH_3$ | 0 | 5-$CH_3$ |
| S | F | F | $CO_2CH_3$ | 0 | 5-$CH_3$ |
| O | F | F | $CO_2CH_3$ | 0 | 5-$CH_3$ |
| S | H | Cl | $CO_2CH_3$ | 0 | 5-$CH_3$ |
| O | H | Cl | $CO_2CH_3$ | 0 | 5-$CH_3$ |
| S | F | Cl | $CO_2CH_3$ | 0 | 5-$CH_3$ |
| O | F | Cl | $CO_2CH_3$ | 0 | 5-$CH_3$ |
| S | H | F | $CO_2CH_2CH_3$ | 0 | 5-$CH_3$ |
| O | H | F | $CO_2CH_2CH_3$ | 0 | 5-$CH_3$ |
| S | F | F | $CO_2CH_2CH_3$ | 0 | 5-$CH_3$ |
| O | F | F | $CO_2CH_2CH_3$ | 0 | 5-$CH_3$ |
| S | H | Cl | $CO_2CH_2CH_3$ | 0 | 5-$CH_3$ |
| O | H | Cl | $CO_2CH_2CH_3$ | 0 | 5-$CH_3$ |
| S | F | Cl | $CO_2CH_2CH_3$ | 0 | 5-$CH_3$ |
| O | F | Cl | $CO_2CH_2CH_3$ | 0 | 5-$CH_3$ |

| X | $R^1$ | $R^2$ | $R^3$ | m | $R^4$ |
|---|---|---|---|---|---|
| S | H | F | $CO_2(CH_2)_2CH_3$ | 0 | 5-$CH_3$ |
| O | H | F | $CO_2(CH_2)_2CH_3$ | 0 | 5-$CH_3$ |
| S | F | F | $CO_2(CH_2)_2CH_3$ | 0 | 5-$CH_3$ |
| O | F | F | $CO_2(CH_2)_2CH_3$ | 0 | 5-$CH_3$ |
| S | H | Cl | $CO_2(CH_2)_2CH_3$ | 0 | 5-$CH_3$ |
| O | H | Cl | $CO_2(CH_2)_2CH_3$ | 0 | 5-$CH_3$ |
| S | F | Cl | $CO_2(CH_2)_2CH_3$ | 0 | 5-$CH_3$ |
| O | F | Cl | $CO_2(CH_2)_2CH_3$ | 0 | 5-$CH_3$ |
| S | H | F | $CO_2CH(CH_3)_2$ | 0 | 5-$CH_3$ |
| O | H | F | $CO_2CH(CH_3)_2$ | 0 | 5-$CH_3$ |
| S | F | F | $CO_2CH(CH_3)_2$ | 0 | 5-$CH_3$ |
| O | F | F | $CO_2CH(CH_3)_2$ | 0 | 5-$CH_3$ |
| S | H | Cl | $CO_2CH(CH_3)_2$ | 0 | 5-$CH_3$ |
| O | H | Cl | $CO_2CH(CH_3)_2$ | 0 | 5-$CH_3$ |
| S | F | Cl | $CO_2CH(CH_3)_2$ | 0 | 5-$CH_3$ |
| O | F | Cl | $CO_2CH(CH_3)_2$ | 0 | 5-$CH_3$ |

| X | $R^1$ | $R^2$ | $R^3$ | m | $R^4$ |
|---|---|---|---|---|---|
| S | H | F | $CO_2(CH_2)_3CH_3$ | 0 | 5-$CH_3$ |
| O | H | F | $CO_2(CH_2)_3CH_3$ | 0 | 5-$CH_3$ |
| S | F | F | $CO_2(CH_2)_3CH_3$ | 0 | 5-$CH_3$ |
| O | F | F | $CO_2(CH_2)_3CH_3$ | 0 | 5-$CH_3$ |
| S | H | Cl | $CO_2(CH_2)_3CH_3$ | 0 | 5-$CH_3$ |
| O | H | Cl | $CO_2(CH_2)_3CH_3$ | 0 | 5-$CH_3$ |
| S | F | Cl | $CO_2(CH_2)_3CH_3$ | 0 | 5-$CH_3$ |
| O | F | Cl | $CO_2(CH_2)_3CH_3$ | 0 | 5-$CH_3$ |
| S | H | F | $CO_2(CH_2)_2OCH_3$ | 0 | 5-$CH_3$ |
| O | H | F | $CO_2(CH_2)_2OCH_3$ | 0 | 5-$CH_3$ |
| S | F | F | $CO_2(CH_2)_2OCH_3$ | 0 | 5-$CH_3$ |
| O | F | F | $CO_2(CH_2)_2OCH_3$ | 0 | 5-$CH_3$ |
| S | H | Cl | $CO_2(CH_2)_2OCH_3$ | 0 | 5-$CH_3$ |
| O | H | Cl | $CO_2(CH_2)_2OCH_3$ | 0 | 5-$CH_3$ |
| S | F | Cl | $CO_2(CH_2)_2OCH_3$ | 0 | 5-$CH_3$ |
| O | F | Cl | $CO_2(CH_2)_2OCH_3$ | 0 | 5-$CH_3$ |

| X | $R^1$ | $R^2$ | $R^3$ | m | $R^4$ |
|---|---|---|---|---|---|
| S | H | F | $CO_2(CH_2)_2OCH_2CH_3$ | 0 | 5-$CH_3$ |
| O | H | F | $CO_2(CH_2)_2OCH_2CH_3$ | 0 | 5-$CH_3$ |
| S | F | F | $CO_2(CH_2)_2OCH_2CH_3$ | 0 | 5-$CH_3$ |
| O | F | F | $CO_2(CH_2)_2OCH_2CH_3$ | 0 | 5-$CH_3$ |
| S | H | Cl | $CO_2(CH_2)_2OCH_2CH_3$ | 0 | 5-$CH_3$ |
| O | H | Cl | $CO_2(CH_2)_2OCH_2CH_3$ | 0 | 5-$CH_3$ |
| S | F | Cl | $CO_2(CH_2)_2OCH_2CH_3$ | 0 | 5-$CH_3$ |
| O | F | Cl | $CO_2(CH_2)_2OCH_2CH_3$ | 0 | 5-$CH_3$ |
| S | H | F | $CO_2CH_3$ | 0 | 4,5-Di-$CH_3$ |
| O | H | F | $CO_2CH_3$ | 0 | 4,5-Di-$CH_3$ |
| S | F | F | $CO_2CH_3$ | 0 | 4,5-Di-$CH_3$ |
| O | F | F | $CO_2CH_3$ | 0 | 4,5-Di-$CH_3$ |
| S | H | Cl | $CO_2CH_3$ | 0 | 4,5-Di-$CH_3$ |
| O | H | Cl | $CO_2CH_3$ | 0 | 4,5-Di-$CH_3$ |
| S | F | Cl | $CO_2CH_3$ | 0 | 4,5-Di-$CH_3$ |
| O | F | Cl | $CO_2CH_3$ | 0 | 4,5-Di-$CH_3$ |

| X | $R^1$ | $R^2$ | $R^3$ | m | $R^4$ |
|---|---|---|---|---|---|
| S | H | F | $CO_2CH_2CH_3$ | 0 | 4,5-Di-$CH_3$ |
| O | H | F | $CO_2CH_2CH_3$ | 0 | 4,5-Di-$CH_3$ |
| S | F | F | $CO_2CH_2CH_3$ | 0 | 4,5-Di-$CH_3$ |
| O | F | F | $CO_2CH_2CH_3$ | 0 | 4,5-Di-$CH_3$ |
| S | H | Cl | $CO_2CH_2CH_3$ | 0 | 4,5-Di-$CH_3$ |
| O | H | Cl | $CO_2CH_2CH_3$ | 0 | 4,5-Di-$CH_3$ |
| S | F | Cl | $CO_2CH_2CH_3$ | 0 | 4,5-Di-$CH_3$ |
| O | F | Cl | $CO_2CH_2CH_3$ | 0 | 4,5-Di-$CH_3$ |
| S | H | F | $CO_2(CH_2)_2CH_3$ | 0 | 4,5-Di-$CH_3$ |
| O | H | F | $CO_2(CH_2)_2CH_3$ | 0 | 4,5-Di-$CH_3$ |
| S | F | F | $CO_2(CH_2)_2CH_3$ | 0 | 4,5-Di-$CH_3$ |
| O | F | F | $CO_2(CH_2)_2CH_3$ | 0 | 4,5-Di-$CH_3$ |
| S | H | Cl | $CO_2(CH_2)_2CH_3$ | 0 | 4,5-Di-$CH_3$ |
| O | H | Cl | $CO_2(CH_2)_2CH_3$ | 0 | 4,5-Di-$CH_3$ |
| S | F | Cl | $CO_2(CH_2)_2CH_3$ | 0 | 4,5-Di-$CH_3$ |
| O | F | Cl | $CO_2(CH_2)_2CH_3$ | 0 | 4,5-Di-$CH_3$ |

| X | $R^1$ | $R^2$ | $R^3$ | m | $R^4$ |
|---|---|---|---|---|---|
| S | H | F | $CO_2CH(CH_3)_2$ | 0 | 4,5-Di-$CH_3$ |
| O | H | F | $CO_2CH(CH_3)_2$ | 0 | 4,5-Di-$CH_3$ |
| S | F | F | $CO_2CH(CH_3)_2$ | 0 | 4,5-Di-$CH_3$ |
| O | F | F | $CO_2CH(CH_3)_2$ | 0 | 4,5-Di-$CH_3$ |
| S | H | Cl | $CO_2CH(CH_3)_2$ | 0 | 4,5-Di-$CH_3$ |
| O | H | Cl | $CO_2CH(CH_3)_2$ | 0 | 4,5-Di-$CH_3$ |
| S | F | Cl | $CO_2CH(CH_3)_2$ | 0 | 4,5-Di-$CH_3$ |
| O | F | Cl | $CO_2CH(CH_3)_2$ | 0 | 4,5-Di-$CH_3$ |
| S | H | F | $CO_2(CH_2)_3CH_3$ | 0 | 4,5-Di-$CH_3$ |
| O | H | F | $CO_2(CH_2)_3CH_3$ | 0 | 4,5-Di-$CH_3$ |
| S | F | F | $CO_2(CH_2)_3CH_3$ | 0 | 4,5-Di-$CH_3$ |
| O | F | F | $CO_2(CH_2)_3CH_3$ | 0 | 4,5-Di-$CH_3$ |
| S | H | Cl | $CO_2(CH_2)_3CH_3$ | 0 | 4,5-Di-$CH_3$ |
| O | H | Cl | $CO_2(CH_2)_3CH_3$ | 0 | 4,5-Di-$CH_3$ |
| S | F | Cl | $CO_2(CH_2)_3CH_3$ | 0 | 4,5-Di-$CH_3$ |
| O | F | Cl | $CO_2(CH_2)_3CH_3$ | 0 | 4,5-Di-$CH_3$ |

| X | $R^1$ | $R^2$ | $R^3$ | m | $R^4$ |
|---|---|---|---|---|---|
| S | H | F | $CO_2(CH_2)_2OCH_3$ | 0 | 4,5-Di-$CH_3$ |
| O | H | F | $CO_2(CH_2)_2OCH_3$ | 0 | 4,5-Di-$CH_3$ |
| S | F | F | $CO_2(CH_2)_2OCH_3$ | 0 | 4,5-Di-$CH_3$ |
| O | F | F | $CO_2(CH_2)_2OCH_3$ | 0 | 4,5-Di-$CH_3$ |
| S | H | Cl | $CO_2(CH_2)_2OCH_3$ | 0 | 4,5-Di-$CH_3$ |
| O | H | Cl | $CO_2(CH_2)_2OCH_3$ | 0 | 4,5-Di-$CH_3$ |
| S | F | Cl | $CO_2(CH_2)_2OCH_3$ | 0 | 4,5-Di-$CH_3$ |
| O | F | Cl | $CO_2(CH_2)_2OCH_3$ | 0 | 4,5-Di-$CH_3$ |
| S | H | F | $CO_2(CH_2)_2OCH_2CH_3$ | 0 | 4,5-Di-$CH_3$ |
| O | H | F | $CO_2(CH_2)_2OCH_2CH_3$ | 0 | 4,5-Di-$CH_3$ |
| S | F | F | $CO_2(CH_2)_2OCH_2CH_3$ | 0 | 4,5-Di-$CH_3$ |
| O | F | F | $CO_2(CH_2)_2OCH_2CH_3$ | 0 | 4,5-Di-$CH_3$ |
| S | H | Cl | $CO_2(CH_2)_2OCH_2CH_3$ | 0 | 4,5-Di-$CH_3$ |
| O | H | Cl | $CO_2(CH_2)_2OCH_2CH_3$ | 0 | 4,5-Di-$CH_3$ |
| S | F | Cl | $CO_2(CH_2)_2OCH_2CH_3$ | 0 | 4,5-Di-$CH_3$ |
| O | F | Cl | $CO_2(CH_2)_2OCH_2CH_3$ | 0 | 4,5-Di-$CH_3$ |

| X | $R^1$ | $R^2$ | $R^3$ | m | $R^4$ |
|---|---|---|---|---|---|
| S | H | F | $CO_2CH_3$ | 1 | H |
| O | H | F | $CO_2CH_3$ | 1 | H |
| S | F | F | $CO_2CH_3$ | 1 | H |
| O | F | F | $CO_2CH_3$ | 1 | H |
| S | H | Cl | $CO_2CH_3$ | 1 | H |
| O | H | Cl | $CO_2CH_3$ | 1 | H |
| S | F | Cl | $CO_2CH_3$ | 1 | H |
| O | F | Cl | $CO_2CH_3$ | 1 | H |
| S | H | F | $CO_2CH_2CH_3$ | 1 | H |
| O | H | F | $CO_2CH_2CH_3$ | 1 | H |
| S | F | F | $CO_2CH_2CH_3$ | 1 | H |
| O | F | F | $CO_2CH_2CH_3$ | 1 | H |
| S | H | Cl | $CO_2CH_2CH_3$ | 1 | H |
| O | H | Cl | $CO_2CH_2CH_3$ | 1 | H |
| S | F | Cl | $CO_2CH_2CH_3$ | 1 | H |
| O | F | Cl | $CO_2CH_2CH_3$ | 1 | H |

| X | $R^1$ | $R^2$ | $R^3$ | m | $R^4$ |
|---|---|---|---|---|---|
| S | H | F | $CO_2(CH_2)_2CH_3$ | 1 | H |
| O | H | F | $CO_2(CH_2)_2CH_3$ | 1 | H |
| S | F | F | $CO_2(CH_2)_2CH_3$ | 1 | H |
| O | F | F | $CO_2(CH_2)_2CH_3$ | 1 | H |
| S | H | Cl | $CO_2(CH_2)_2CH_3$ | 1 | H |
| O | H | Cl | $CO_2(CH_2)_2CH_3$ | 1 | H |
| S | F | Cl | $CO_2(CH_2)_2CH_3$ | 1 | H |
| O | F | Cl | $CO_2(CH_2)_2CH_3$ | 1 | H |
| S | H | F | $CO_2CH(CH_3)_2$ | 1 | H |
| O | H | F | $CO_2CH(CH_3)_2$ | 1 | H |
| S | F | F | $CO_2CH(CH_3)_2$ | 1 | H |
| O | F | F | $CO_2CH(CH_3)_2$ | 1 | H |
| S | H | Cl | $CO_2CH(CH_3)_2$ | 1 | H |
| O | H | Cl | $CO_2CH(CH_3)_2$ | 1 | H |
| S | F | Cl | $CO_2CH(CH_3)_2$ | 1 | H |
| O | F | Cl | $CO_2CH(CH_3)_2$ | 1 | H |

| X | $R^1$ | $R^2$ | $R^3$ | m | $R^4$ |
|---|---|---|---|---|---|
| S | H | F | $CO_2(CH_2)_3CH_3$ | 1 | H |
| O | H | F | $CO_2(CH_2)_3CH_3$ | 1 | H |
| S | F | F | $CO_2(CH_2)_3CH_3$ | 1 | H |
| O | F | F | $CO_2(CH_2)_3CH_3$ | 1 | H |
| S | H | Cl | $CO_2(CH_2)_3CH_3$ | 1 | H |
| O | H | Cl | $CO_2(CH_2)_3CH_3$ | 1 | H |
| S | F | Cl | $CO_2(CH_2)_3CH_3$ | 1 | H |
| O | F | Cl | $CO_2(CH_2)_3CH_3$ | 1 | H |
| S | H | F | $CO_2(CH_2)_2OCH_3$ | 1 | H |
| O | H | F | $CO_2(CH_2)_2OCH_3$ | 1 | H |
| S | F | F | $CO_2(CH_2)_2OCH_3$ | 1 | H |
| O | F | F | $CO_2(CH_2)_2OCH_3$ | 1 | H |
| S | H | Cl | $CO_2(CH_2)_2OCH_3$ | 1 | H |
| O | H | Cl | $CO_2(CH_2)_2OCH_3$ | 1 | H |
| S | F | Cl | $CO_2(CH_2)_2OCH_3$ | 1 | H |
| O | F | Cl | $CO_2(CH_2)_2OCH_3$ | 1 | H |

| X | $R^1$ | $R^2$ | $R^3$ | m | $R^4$ |
|---|---|---|---|---|---|
| S | H | F | $CO_2(CH_2)_2OCH_2CH_3$ | 1 | H |
| O | H | F | $CO_2(CH_2)_2OCH_2CH_3$ | 1 | H |
| S | F | F | $CO_2(CH_2)_2OCH_2CH_3$ | 1 | H |
| O | F | F | $CO_2(CH_2)_2OCH_2CH_3$ | 1 | H |
| S | H | Cl | $CO_2(CH_2)_2OCH_2CH_3$ | 1 | H |
| O | H | Cl | $CO_2(CH_2)_2OCH_2CH_3$ | 1 | H |
| S | F | Cl | $CO_2(CH_2)_2OCH_2CH_3$ | 1 | H |
| O | F | Cl | $CO_2(CH_2)_2OCH_2CH_3$ | 1 | H |
| S | H | F | $CO_2CH_3$ | 1 | $4-CH_3$ |
| O | H | F | $CO_2CH_3$ | 1 | $4-CH_3$ |
| S | F | F | $CO_2CH_3$ | 1 | $4-CH_3$ |
| O | F | F | $CO_2CH_3$ | 1 | $4-CH_3$ |
| S | H | Cl | $CO_2CH_3$ | 1 | $4-CH_3$ |
| O | H | Cl | $CO_2CH_3$ | 1 | $4-CH_3$ |
| S | F | Cl | $CO_2CH_3$ | 1 | $4-CH_3$ |
| O | F | Cl | $CO_2CH_3$ | 1 | $4-CH_3$ |

| X | $R^1$ | $R^2$ | $R^3$ | m | $R^4$ |
|---|---|---|---|---|---|
| S | H | F | $CO_2CH_2CH_3$ | 1 | $4-CH_3$ |
| O | H | F | $CO_2CH_2CH_3$ | 1 | $4-CH_3$ |
| S | F | F | $CO_2CH_2CH_3$ | 1 | $4-CH_3$ |
| O | F | F | $CO_2CH_2CH_3$ | 1 | $4-CH_3$ |
| S | H | Cl | $CO_2CH_2CH_3$ | 1 | $4-CH_3$ |
| O | H | Cl | $CO_2CH_2CH_3$ | 1 | $4-CH_3$ |
| S | F | Cl | $CO_2CH_2CH_3$ | 1 | $4-CH_3$ |
| O | F | Cl | $CO_2CH_2CH_3$ | 1 | $4-CH_3$ |
| S | H | F | $CO_2(CH_2)_2CH_3$ | 1 | $4-CH_3$ |
| O | H | F | $CO_2(CH_2)_2CH_3$ | 1 | $4-CH_3$ |
| S | F | F | $CO_2(CH_2)_2CH_3$ | 1 | $4-CH_3$ |
| O | F | F | $CO_2(CH_2)_2CH_3$ | 1 | $4-CH_3$ |
| S | H | Cl | $CO_2(CH_2)_2CH_3$ | 1 | $4-CH_3$ |
| O | H | Cl | $CO_2(CH_2)_2CH_3$ | 1 | $4-CH_3$ |
| S | F | Cl | $CO_2(CH_2)_2CH_3$ | 1 | $4-CH_3$ |
| O | F | Cl | $CO_2(CH_2)_2CH_3$ | 1 | $4-CH_3$ |

| X | $R^1$ | $R^2$ | $R^3$ | m | $R^4$ |
|---|---|---|---|---|---|
| S | H | F | $CO_2CH(CH_3)_2$ | 1 | 4-$CH_3$ |
| O | H | F | $CO_2CH(CH_3)_2$ | 1 | 4-$CH_3$ |
| S | F | F | $CO_2CH(CH_3)_2$ | 1 | 4-$CH_3$ |
| O | F | F | $CO_2CH(CH_3)_2$ | 1 | 4-$CH_3$ |
| S | H | Cl | $CO_2CH(CH_3)_2$ | 1 | 4-$CH_3$ |
| O | H | Cl | $CO_2CH(CH_3)_2$ | 1 | 4-$CH_3$ |
| S | F | Cl | $CO_2CH(CH_3)_2$ | 1 | 4-$CH_3$ |
| O | F | Cl | $CO_2CH(CH_3)_2$ | 1 | 4-$CH_3$ |
| S | H | F | $CO_2(CH_2)_3CH_3$ | 1 | 4-$CH_3$ |
| O | H | F | $CO_2(CH_2)_3CH_3$ | 1 | 4-$CH_3$ |
| S | F | F | $CO_2(CH_2)_3CH_3$ | 1 | 4-$CH_3$ |
| O | F | F | $CO_2(CH_2)_3CH_3$ | 1 | 4-$CH_3$ |
| S | H | Cl | $CO_2(CH_2)_3CH_3$ | 1 | 4-$CH_3$ |
| O | H | Cl | $CO_2(CH_2)_3CH_3$ | 1 | 4-$CH_3$ |
| S | F | Cl | $CO_2(CH_2)_3CH_3$ | 1 | 4-$CH_3$ |
| O | F | Cl | $CO_2(CH_2)_3CH_3$ | 1 | 4-$CH_3$ |

| X | $R^1$ | $R^2$ | $R^3$ | m | $R^4$ |
|---|---|---|---|---|---|
| S | H | F | $CO_2(CH_2)_2OCH_3$ | 1 | 4-$CH_3$ |
| O | H | F | $CO_2(CH_2)_2OCH_3$ | 1 | 4-$CH_3$ |
| S | F | F | $CO_2(CH_2)_2OCH_3$ | 1 | 4-$CH_3$ |
| O | F | F | $CO_2(CH_2)_2OCH_3$ | 1 | 4-$CH_3$ |
| S | H | Cl | $CO_2(CH_2)_2OCH_3$ | 1 | 4-$CH_3$ |
| O | H | Cl | $CO_2(CH_2)_2OCH_3$ | 1 | 4-$CH_3$ |
| S | F | Cl | $CO_2(CH_2)_2OCH_3$ | 1 | 4-$CH_3$ |
| O | F | Cl | $CO_2(CH_2)_2OCH_3$ | 1 | 4-$CH_3$ |
| S | H | F | $CO_2(CH_2)_2OCH_2CH_3$ | 1 | 4-$CH_3$ |
| O | H | F | $CO_2(CH_2)_2OCH_2CH_3$ | 1 | 4-$CH_3$ |
| S | F | F | $CO_2(CH_2)_2OCH_2CH_3$ | 1 | 4-$CH_3$ |
| O | F | F | $CO_2(CH_2)_2OCH_2CH_3$ | 1 | 4-$CH_3$ |
| S | H | Cl | $CO_2(CH_2)_2OCH_2CH_3$ | 1 | 4-$CH_3$ |
| O | H | Cl | $CO_2(CH_2)_2OCH_2CH_3$ | 1 | 4-$CH_3$ |
| S | F | Cl | $CO_2(CH_2)_2OCH_2CH_3$ | 1 | 4-$CH_3$ |
| O | F | Cl | $CO_2(CH_2)_2OCH_2CH_3$ | 1 | 4-$CH_3$ |

| X | $R^1$ | $R^2$ | $R^3$ | m | $R^4$ |
|---|---|---|---|---|---|
| S | H | F | $CO_2CH_3$ | 1 | $6-CH_3$ |
| O | H | F | $CO_2CH_3$ | 1 | $6-CH_3$ |
| S | F | F | $CO_2CH_3$ | 1 | $6-CH_3$ |
| O | F | F | $CO_2CH_3$ | 1 | $6-CH_3$ |
| S | H | Cl | $CO_2CH_3$ | 1 | $6-CH_3$ |
| O | H | Cl | $CO_2CH_3$ | 1 | $6-CH_3$ |
| S | F | Cl | $CO_2CH_3$ | 1 | $6-CH_3$ |
| O | F | Cl | $CO_2CH_3$ | 1 | $6-CH_3$ |
| S | H | F | $CO_2CH_2CH_3$ | 1 | $6-CH_3$ |
| O | H | F | $CO_2CH_2CH_3$ | 1 | $6-CH_3$ |
| S | F | F | $CO_2CH_2CH_3$ | 1 | $6-CH_3$ |
| O | F | F | $CO_2CH_2CH_3$ | 1 | $6-CH_3$ |
| S | H | Cl | $CO_2CH_2CH_3$ | 1 | $6-CH_3$ |
| O | H | Cl | $CO_2CH_2CH_3$ | 1 | $6-CH_3$ |
| S | F | Cl | $CO_2CH_2CH_3$ | 1 | $6-CH_3$ |
| O | F | Cl | $CO_2CH_2CH_3$ | 1 | $6-CH_3$ |

| X | $R^1$ | $R^2$ | $R^3$ | m | $R^4$ |
|---|---|---|---|---|---|
| S | H | F | $CO_2(CH_2)_2CH_3$ | 1 | $6-CH_3$ |
| O | H | F | $CO_2(CH_2)_2CH_3$ | 1 | $6-CH_3$ |
| S | F | F | $CO_2(CH_2)_2CH_3$ | 1 | $6-CH_3$ |
| O | F | F | $CO_2(CH_2)_2CH_3$ | 1 | $6-CH_3$ |
| S | H | Cl | $CO_2(CH_2)_2CH_3$ | 1 | $6-CH_3$ |
| O | H | Cl | $CO_2(CH_2)_2CH_3$ | 1 | $6-CH_3$ |
| S | F | Cl | $CO_2(CH_2)_2CH_3$ | 1 | $6-CH_3$ |
| O | F | Cl | $CO_2(CH_2)_2CH_3$ | 1 | $6-CH_3$ |
| S | H | F | $CO_2CH(CH_3)_2$ | 1 | $6-CH_3$ |
| O | H | F | $CO_2CH(CH_3)_2$ | 1 | $6-CH_3$ |
| S | F | F | $CO_2CH(CH_3)_2$ | 1 | $6-CH_3$ |
| O | F | F | $CO_2CH(CH_3)_2$ | 1 | $6-CH_3$ |
| S | H | Cl | $CO_2CH(CH_3)_2$ | 1 | $6-CH_3$ |
| O | H | Cl | $CO_2CH(CH_3)_2$ | 1 | $6-CH_3$ |
| S | F | Cl | $CO_2CH(CH_3)_2$ | 1 | $6-CH_3$ |
| O | F | Cl | $CO_2CH(CH_3)_2$ | 1 | $6-CH_3$ |

| X | $R^1$ | $R^2$ | $R^3$ | m | $R^4$ |
|---|---|---|---|---|---|
| S | H | F | $CO_2(CH_2)_3CH_3$ | 1 | 6-$CH_3$ |
| O | H | F | $CO_2(CH_2)_3CH_3$ | 1 | 6-$CH_3$ |
| S | F | F | $CO_2(CH_2)_3CH_3$ | 1 | 6-$CH_3$ |
| O | F | F | $CO_2(CH_2)_3CH_3$ | 1 | 6-$CH_3$ |
| S | H | Cl | $CO_2(CH_2)_3CH_3$ | 1 | 6-$CH_3$ |
| O | H | Cl | $CO_2(CH_2)_3CH_3$ | 1 | 6-$CH_3$ |
| S | F | Cl | $CO_2(CH_2)_3CH_3$ | 1 | 6-$CH_3$ |
| O | F | Cl | $CO_2(CH_2)_3CH_3$ | 1 | 6-$CH_3$ |
| S | H | F | $CO_2(CH_2)_2OCH_3$ | 1 | 6-$CH_3$ |
| O | H | F | $CO_2(CH_2)_2OCH_3$ | 1 | 6-$CH_3$ |
| S | F | F | $CO_2(CH_2)_2OCH_3$ | 1 | 6-$CH_3$ |
| O | F | F | $CO_2(CH_2)_2OCH_3$ | 1 | 6-$CH_3$ |
| S | H | Cl | $CO_2(CH_2)_2OCH_3$ | 1 | 6-$CH_3$ |
| O | H | Cl | $CO_2(CH_2)_2OCH_3$ | 1 | 6-$CH_3$ |
| S | F | Cl | $CO_2(CH_2)_2OCH_3$ | 1 | 6-$CH_3$ |
| O | F | Cl | $CO_2(CH_2)_2OCH_3$ | 1 | 6-$CH_3$ |

| X | $R^1$ | $R^2$ | $R^3$ | m | $R^4$ |
|---|---|---|---|---|---|
| S | H | F | $CO_2(CH_2)_2OCH_2CH_3$ | 1 | 6-$CH_3$ |
| O | H | F | $CO_2(CH_2)_2OCH_2CH_3$ | 1 | 6-$CH_3$ |
| S | F | F | $CO_2(CH_2)_2OCH_2CH_3$ | 1 | 6-$CH_3$ |
| O | F | F | $CO_2(CH_2)_2OCH_2CH_3$ | 1 | 6-$CH_3$ |
| S | H | Cl | $CO_2(CH_2)_2OCH_2CH_3$ | 1 | 6-$CH_3$ |
| O | H | Cl | $CO_2(CH_2)_2OCH_2CH_3$ | 1 | 6-$CH_3$ |
| S | F | Cl | $CO_2(CH_2)_2OCH_2CH_3$ | 1 | 6-$CH_3$ |
| O | F | Cl | $CO_2(CH_2)_2OCH_2CH_3$ | 1 | 6-$CH_3$ |
| S | H | F | $CO_2CH_3$ | 1 | 4,6-Di-$CH_3$ |
| O | H | F | $CO_2CH_3$ | 1 | 4,6-Di-$CH_3$ |
| S | F | F | $CO_2CH_3$ | 1 | 4,6-Di-$CH_3$ |
| O | F | F | $CO_2CH_3$ | 1 | 4,6-Di-$CH_3$ |
| S | H | Cl | $CO_2CH_3$ | 1 | 4,6-Di-$CH_3$ |
| O | H | Cl | $CO_2CH_3$ | 1 | 4,6-Di-$CH_3$ |
| S | F | Cl | $CO_2CH_3$ | 1 | 4,6-Di-$CH_3$ |
| O | F | Cl | $CO_2CH_3$ | 1 | 4,6-Di-$CH_3$ |

| X | $R^1$ | $R^2$ | $R^3$ | m | $R^4$ |
|---|---|---|---|---|---|
| S | H | F | $CO_2CH_2CH_3$ | 1 | 4,6-Di-$CH_3$ |
| O | H | F | $CO_2CH_2CH_3$ | 1 | 4,6-Di-$CH_3$ |
| S | F | F | $CO_2CH_2CH_3$ | 1 | 4,6-Di-$CH_3$ |
| O | F | F | $CO_2CH_2CH_3$ | 1 | 4,6-Di-$CH_3$ |
| S | H | Cl | $CO_2CH_2CH_3$ | 1 | 4,6-Di-$CH_3$ |
| O | H | Cl | $CO_2CH_2CH_3$ | 1 | 4,6-Di-$CH_3$ |
| S | F | Cl | $CO_2CH_2CH_3$ | 1 | 4,6-Di-$CH_3$ |
| O | F | Cl | $CO_2CH_2CH_3$ | 1 | 4,6-Di-$CH_3$ |
| S | H | F | $CO_2(CH_2)_2CH_3$ | 1 | 4,6-Di-$CH_3$ |
| O | H | F | $CO_2(CH_2)_2CH_3$ | 1 | 4,6-Di-$CH_3$ |
| S | F | F | $CO_2(CH_2)_2CH_3$ | 1 | 4,6-Di-$CH_3$ |
| O | F | F | $CO_2(CH_2)_2CH_3$ | 1 | 4,6-Di-$CH_3$ |
| S | H | Cl | $CO_2(CH_2)_2CH_3$ | 1 | 4,6-Di-$CH_3$ |
| O | H | Cl | $CO_2(CH_2)_2CH_3$ | 1 | 4,6-Di-$CH_3$ |
| S | F | Cl | $CO_2(CH_2)_2CH_3$ | 1 | 4,6-Di-$CH_3$ |
| O | F | Cl | $CO_2(CH_2)_2CH_3$ | 1 | 4,6-Di-$CH_3$ |

| X | $R^1$ | $R^2$ | $R^3$ | m | $R^4$ |
|---|---|---|---|---|---|
| S | H | F | $CO_2CH(CH_3)_2$ | 1 | 4,6-Di-$CH_3$ |
| O | H | F | $CO_2CH(CH_3)_2$ | 1 | 4,6-Di-$CH_3$ |
| S | F | F | $CO_2CH(CH_3)_2$ | 1 | 4,6-Di-$CH_3$ |
| O | F | F | $CO_2CH(CH_3)_2$ | 1 | 4,6-Di-$CH_3$ |
| S | H | Cl | $CO_2CH(CH_3)_2$ | 1 | 4,6-Di-$CH_3$ |
| O | H | Cl | $CO_2CH(CH_3)_2$ | 1 | 4,6-Di-$CH_3$ |
| S | F | Cl | $CO_2CH(CH_3)_2$ | 1 | 4,6-Di-$CH_3$ |
| O | F | Cl | $CO_2CH(CH_3)_2$ | 1 | 4,6-Di-$CH_3$ |
| S | H | F | $CO_2(CH_2)_3CH_3$ | 1 | 4,6-Di-$CH_3$ |
| O | H | F | $CO_2(CH_2)_3CH_3$ | 1 | 4,6-Di-$CH_3$ |
| S | F | F | $CO_2(CH_2)_3CH_3$ | 1 | 4,6-Di-$CH_3$ |
| O | F | F | $CO_2(CH_2)_3CH_3$ | 1 | 4,6-Di-$CH_3$ |
| S | H | Cl | $CO_2(CH_2)_3CH_3$ | 1 | 4,6-Di-$CH_3$ |
| O | H | Cl | $CO_2(CH_2)_3CH_3$ | 1 | 4,6-Di-$CH_3$ |
| S | F | Cl | $CO_2(CH_2)_3CH_3$ | 1 | 4,6-Di-$CH_3$ |
| O | F | Cl | $CO_2(CH_2)_3CH_3$ | 1 | 4,6-Di-$CH_3$ |

| X | $R^1$ | $R^2$ | $R^3$ | m | $R^4$ |
|---|---|---|---|---|---|
| O | H | F | $CO_2(CH_2)_2OCH_3$ | 1 | 4,6-Di-$CH_3$ |
| S | F | F | $CO_2(CH_2)_2OCH_3$ | 1 | 4,6-Di-$CH_3$ |
| O | F | F | $CO_2(CH_2)_2OCH_3$ | 1 | 4,6-Di-$CH_3$ |
| S | H | Cl | $CO_2(CH_2)_2OCH_3$ | 1 | 4,6-Di-$CH_3$ |
| O | H | Cl | $CO_2(CH_2)_2OCH_3$ | 1 | 4,6-Di-$CH_3$ |
| S | F | Cl | $CO_2(CH_2)_2OCH_3$ | 1 | 4,6-Di-$CH_3$ |
| O | F | Cl | $CO_2(CH_2)_2OCH_3$ | 1 | 4,6-Di-$CH_3$ |
| S | H | F | $CO_2(CH_2)_2OCH_2CH_3$ | 1 | 4,6-Di-$CH_3$ |
| O | H | F | $CO_2(CH_2)_2OCH_2CH_3$ | 1 | 4,6-Di-$CH_3$ |
| S | F | F | $CO_2(CH_2)_2OCH_2CH_3$ | 1 | 4,6-Di-$CH_3$ |
| O | F | F | $CO_2(CH_2)_2OCH_2CH_3$ | 1 | 6-$CH_3$ |
| S | H | Cl | $CO_2(CH_2)_2OCH_2CH_3$ | 1 | 6-$CH_3$ |
| O | H | Cl | $CO_2(CH_2)_2OCH_2CH_3$ | 1 | 6-$CH_3$ |
| S | F | Cl | $CO_2(CH_2)_2OCH_2CH_3$ | 1 | 6-$CH_3$ |
| O | F | Cl | $CO_2(CH_2)_2OCH_2CH_3$ | 1 | 6-$CH_3$ |

Tabelle 2

| X | $R^1$ | $R^2$ | $R^3$ | m | $R^4$ |
|---|---|---|---|---|---|
| S | H | F | H | 0 | H |
| O | H | F | H | 0 | H |
| S | F | F | H | 0 | H |
| O | F | F | H | 0 | H |
| S | H | Cl | H | 0 | H |
| O | H | Cl | H | 0 | H |
| S | F | Cl | H | 0 | H |
| O | F | Cl | H | 0 | H |
| S | H | F | H | 0 | 4-$CH_3$ |
| O | H | F | H | 0 | 4-$CH_3$ |
| S | F | F | H | 0 | 4-$CH_3$ |
| O | F | F | H | 0 | 4-$CH_3$ |
| S | H | Cl | H | 0 | 4-$CH_3$ |
| O | H | Cl | H | 0 | 4-$CH_3$ |
| S | F | Cl | H | 0 | 4-$CH_3$ |
| O | F | Cl | H | 0 | 4-$CH_3$ |

| X | $R^1$ | $R^2$ | $R^3$ | m | $R^4$ |
|---|---|---|---|---|---|
| S | H | F | H | 0 | 4,5-Di-$CH_3$ |
| O | H | F | H | 0 | 4,5-Di-$CH_3$ |
| S | F | F | H | 0 | 4,5-Di-$CH_3$ |
| O | F | F | H | 0 | 4,5-Di-$CH_3$ |
| S | H | Cl | H | 0 | 4,5-Di-$CH_3$ |
| O | H | Cl | H | 0 | 4,5-Di-$CH_3$ |
| S | F | Cl | H | 0 | 4,5-Di-$CH_3$ |
| O | F | Cl | H | 0 | 4,5-Di-$CH_3$ |
| S | H | F | H | 0 | 4-$CH_2CH_3$ |
| O | H | F | H | 0 | 4-$CH_2CH_3$ |
| S | F | F | H | 0 | 4-$CH_2CH_3$ |
| O | F | F | H | 0 | 4-$CH_2CH_3$ |
| S | H | Cl | H | 0 | 4-$CH_2CH_3$ |
| O | H | Cl | H | 0 | 4-$CH_2CH_3$ |
| S | F | Cl | H | 0 | 4-$CH_2CH_3$ |
| O | F | Cl | H | 0 | 4-$CH_2CH_3$ |

| X | $R^1$ | $R^2$ | $R^3$ | m | $R^4$ |
|---|---|---|---|---|---|
| S | H | F | H | 1 | H |
| O | H | F | H | 1 | H |
| S | F | F | H | 1 | H |
| O | F | F | H | 1 | H |
| S | H | Cl | H | 1 | H |
| O | H | Cl | H | 1 | H |
| S | F | Cl | H | 1 | H |
| O | F | Cl | H | 1 | H |
| S | H | F | H | 1 | 4-$CH_3$ |
| O | H | F | H | 1 | 4-$CH_3$ |
| S | F | F | H | 1 | 4-$CH_3$ |
| O | F | F | H | 1 | 4-$CH_3$ |
| S | H | Cl | H | 1 | 4-$CH_3$ |
| O | H | Cl | H | 1 | 4-$CH_3$ |
| S | F | Cl | H | 1 | 4-$CH_3$ |
| O | F | Cl | H | 1 | 4-$CH_3$ |

| X | $R^1$ | $R^2$ | $R^3$ | m | $R^4$ |
|---|---|---|---|---|---|
| S | H | F | H | 1 | 4,6-Di-$CH_3$ |
| O | H | F | H | 1 | 4,6-Di-$CH_3$ |
| S | F | F | H | 1 | 4,6-Di-$CH_3$ |
| O | F | F | H | 1 | 4,6-Di-$CH_3$ |
| S | H | Cl | H | 1 | 4,6-Di-$CH_3$ |
| O | H | Cl | H | 1 | 4,6-Di-$CH_3$ |
| S | F | Cl | H | 1 | 4,6-Di-$CH_3$ |
| O | F | Cl | H | 1 | 4,6-Di-$CH_3$ |
| S | H | F | H | 1 | 4-$CH_2CH_3$ |
| O | H | F | H | 1 | 4-$CH_2CH_3$ |
| S | F | F | H | 1 | 4-$CH_2CH_3$ |
| O | F | F | H | 1 | 4-$CH_2CH_3$ |
| S | H | Cl | H | 1 | 4-$CH_2CH_3$ |
| O | H | Cl | H | 1 | 4-$CH_2CH_3$ |
| S | F | Cl | H | 1 | 4-$CH_2CH_3$ |
| O | F | Cl | H | 1 | 4-$CH_2CH_3$ |

Die Thiadiaza-bicyclononan-Derivate I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen,

Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Beispiele für solche Zubereitungen sind:

I. eine Lösung aus 90 Gew.-Teilen der Verbindung Nr. 3.01 und 10 Gew.-Teilen N-Methyl-α-pyrrolidon, die zur Anwendung in Form kleinster Tropfen geeignet ist;

II. eine Mischung aus 20 Gew.-Teilen der Verbindung Nr. 3.02, 80 Gew.Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes und 40 Mol Ethylenoxid an 1 Mol Ricinusöl; durch feines Verteilen der Lösung in Wasser erhält man eine Dispersion.

III. eine wäßrige Dispersion aus 20 Gew.-Teilen der Verbindung Nr. 3.03, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;

IV. eine wäßrige Dispersion aus 20 Gew.-Teilen der Verbindung Nr. 3.04, 25 Gew.-Teilen Cyclohexanon, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl;

V. eine in einer Hammermühle vermahlene Mischung aus 20 Gew.-Teilen der Verbindung Nr. 3.05, 3 Gew.-Teilen des Natriumsalzes der Diisobutylnaphtalin-α-sulfonsäure, 17 Gew.-Teilen des Natriumsalzes einer Ligninsulfonsäure aus einer Sulfitablauge und 60 Gew.-Teilen pulverförmigem Kieselsäuregel; durch feines Verteilen der Mischung in 20.000 Gew.-Teilen Wasser erhält man eine Spritzbrühe, die 0,1 Gew.-% des Wirkstoffs enthält.

VI. eine innige Mischung aus 3 Gew.-Teilen der Verbindung Nr. 3.07 und 97 Gew.-Teilen feinteiligem Kaolin; dieses Stäubemittel enthält 3 Gew.-% Wirkstoff;

VII. eine innige Mischung aus 30 Gew.-Teilen der Verbindung Nr. 3.11, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;

VIII. eine stabile ölige Dispersion aus 20 Gew.-Teilen der Verbindung Nr. 3.12, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoffformaldehydKondensates und 68 Gew.-Teilen eines paraffinischen Mineralöls.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

In Anbetracht der Vielseitigkeit der Applikationsmethoden können die erfindungsgemäßen Verbindungen bzw. sie enthaltende Mittel noch in einer weiteren Zahl von Kulturplfanzen zur Beseitigung unerwünschter Pflanzen eingesetzt werden. In Betracht kommen beispielsweise folgende Kulturen (botanische Namen):
Allium cepa, Ananas comosus, Arachis hypogaea, Asparagus officinalis, Avena sativa, Beta vulgaris spp. altissima, Beta vulgaris spp. rapa, Beta vulgaris spp. esculenta, Brassica napus var. napus, Brassica napus var. napobrassica, Brassica napus war. rapa, Brassica rapa war. silvestris, Camellia sinensis, Carthamus tinctorius, Carya illinoinensis, Citrus limon, Citrus maxima, Citrus reticulata, Citrus sinensis, Coffea arabica (Coffea canephora, Coffea liberica), Cucumis melo, Cucumis sativus, Cynodon dactylon, Daucus carota, Elaeis guineensis, Fragaria vesca, Glycine max, Gossypium hirsutum (Gossypium arboreum, Gossypium herbaceum, Gossypium vitifolium), Helianthus annuus, Helianthus tuberosus, Hevea brasiliensis, Hordeum

vulgare, Humulus lupulus, Ipomoea batatas, Juglans regia, Lactuca sativa, Lens culinaris, Linum usitatissimum, Lycopersicon lycopersicum, Malus spp., Manihut esculenta, Medicago sativa, Mentha piperita, Musa spp., Nicotiana tabacum (N. rustica), Olea europaea, Oryza sativa, Panicum miliaceum, Phaseolus lunatus, Phaseolus mungo, Phaseolus vulgaris, Pennisetum glaucum, Petroselinum crispum spp. tuberosum, Picea abies, Abies alba, Pinus spp., Pisum sativum, Prunus avium, Prunus domestica, Prunus dulcis, Prunus persica, Pyrus communis, Ribes sylvestre, Ribes uva-crispa, Ricinus communis, Saccharum officinarum, Secale cereale, Sesamum indicum, Solanum tuberosum, Sorghum bicolor (s. vulgare), Sorghum dochna, Spinacia oleracea, Theobroma cacao, Trifolium pratense, Triticum aestivum, Triticum durum, Vaccinium corymbosum, Vaccinium vitis-idaea, Vicia faba, Vigna sinensis (V. unguiculata), Vitis winifera, Zea mays.

Zur Verbreiterung des Wirkungsspektrums und zur Erzielung synergistischer Effekte können die Acetale und Thioacetale I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Aryloxy-, Heteroaryloxyphenoxypropionsäuren sowie deren Salze, Ester und Amide und andere in Betracht.

Außerdem kann es von Nutzen sein, die herbiziden Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Synthesebeispiele

Beispiel 1

9-[(4-Methyl-1,3-dithiolan-2-yl)-4-chlorphenylimino]-8-thia-1,6-diazabicyclo-[4.3.0]-nonan-7-on (Verbindung Nr. 3.12 in Tabelle 3)

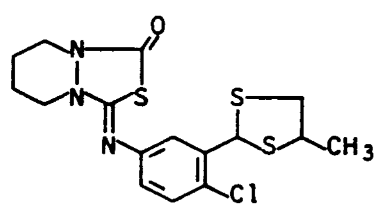

Zu einer Mischung aus 3,7 g (0,01 mol) N-[4-Chlor-3-(3-methyl-1,3-dithiolan-2-yl)-phenylthiocarbamoyl]-hexahydropyridazin, 1,7 g (0,022 mol) Pyridin und 130 ml Methylenchlorid wurden bei 25-30 °C 2,2 g (0,011 mol) Chlorameisensäuretrichlormethylester in 20 ml Methylenchlorid getropft. Danach wurde 2 Stunden bei 25 °C gerührt, 3 mal mit je 50 ml Wasser neutral gewaschen und eingeengt. Das Rohprodukt wurde aus Petrolether umkristallisiert. Ausbeute: 88 %.

Vorstufe 1α)

4-Chlor-3-(4-methyl-1,3-dithiolan-2-yl)-nitrobenzol

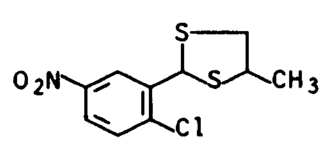

Eine Lösung aus 37,1 g (0,2 mol) 2-Chlor-5-nitro-benzaldehyd, 1 g ($5 \cdot 10^{-3}$ mol) p-Toluolsulfonsäure und 500 ml Toluol wurde mit 22,8 g (0,21 mol) 1,2-Propandithiol versetzt und 5 Stunden lang unter Rückflußkühlung am Wasserabscheider erhitzt. Nach dem Abkühlen wurde das Lösungsmittel entfernt und der Rückstand mit Petrolether gewaschen. Ausbeute: 94 %; Fp.: 55 °C.

Vorstufe 1β)
4-Chlor-3-(4-methyl-1,3-dithiolan-2-yl)-anilin

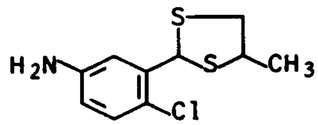

Eine Mischung aus 31,5 g (0,56 mol) Eisenpulver in 100 ml Methanol und 150 ml Eisessig wurde unter Rückflußkühlung mit 51,5 g (0,19 mol) 4-Chlor-3-(4-methyl-1,3-dithiolan-2-yl)-nitrobenzol versetzt und 2 Stunden erhitzt. Nach Einrühren in 400 ml Wasser wurde abgesaugt, das Filtrat mit Essigester extrahiert, die organische Phase mit Wasser gewaschen, getrocknet und eingeengt. Ausbeute: 81 %; Öl.
Vorstufe 1γ)
2-(4-Methyl-1,3-dithiolan-2-yl)-4-isothiocyanato-chlorbenzol

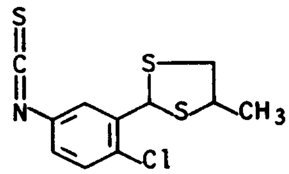

Zu einer Mischung aus 6,3 g (0,055 mol) Thiophosgen, 50 ml Methylenchlorid und 100 ml Wasser wurden bei 25-30 °C 12,3 g (0,05 mol) 4-Chlor-3-(4-methyl)-1,3-dithiolan-2-yl)-anilin in 100 ml Methylenchlorid zugetropft. Nach 2stündigem rühren bei 25 °C wurde die organische Phase abgetrennt, zweimal mit je 50 ml ges. Natriumhydrogencarbonatlösung und Wasser neutral gewaschen, getrocknet und eingeengt. Ausbeute: 90 %; Öl
Vorstufe 1δ)
N-[4-Chlor-3-(3-methyl-1,3-dithiolan-2-yl)-phenylthiocarbamoyl]-hexahydropyridazin;

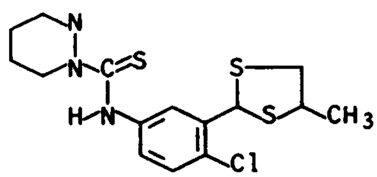

Zu einer Lösung von 4,3 g (0,05 mol) Piperazin in 200 ml Tetrahydrofuran wurden bei 25-30 °C 13 g (0,045 mol) 2-(4-Methyl-1,3-dithiolan-2-yl)-4-isothiocyanato-chlorbenzol in 50 ml Tetrahydrofuran getropft. Nach 2 Stunden bei 25 °C wurde das Lösungsmittel entfernt. Ausbeute: 59 %; Fp.: 124-125 °C.

Beispiel 2

7-[(1,3-Dithian-2-yl)-4-chlorphenylimino]-8-thia-1,6-diazabicyclo-[4.3.0]-non-2-en-9-on (Verbindung Nr. 3.04 in Tabelle 3)

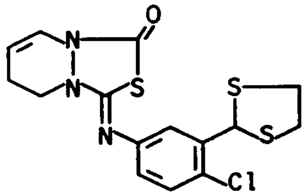

Eine Mischung aus 3,1 g (10 mmol) 7-(2-Formyl-4-chlorphenylimino)-8-thia-1,6-diazabicyclo-[4.3.0]-non-2-en-9-on, 0,1 g (0,5 mmol) p-Toluolsulfonsäure und 200 ml Toluol wurde mit 2 Portionen zu je 1,2 g (11 mmol) 1,3-Propandithiol versetzt und unter Entzug des Reaktionswassers 15 Stunden auf Rückflußtemperatur erhitzt. Danach wurde das Gemisch 2 mal mit jeweils 50 ml 10 gew.-%iger Natronlauge und 3 mal mit Wasser gewaschen und anschließend auf das Produkt hin aufgearbeitet. Ausbeute: 63 %.

Vorstufe 2α)

2-(1,3-Dioxolan-2-yl)-4-isothiocyanato-chlorbenzol

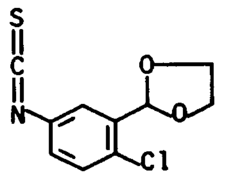

Zu einer Lösung aus 100 g (0,5 mol) 4-Chlor-3-(1,3-dioxolan-2-yl)-anilin und 60 g (0,6 mol) Triethylamin in 1 l Dichlormethan wurden unter Eiskühlung 6,3 g (0,55 mol) Thiophosgen getropft. Nach 2 Stunden Rühren bei 25 °C wurde die Mischung 3 mal mit jeweils 500 ml Wasser gewaschen und dann mit Natriumsulfat getrocknet. Anschließend wurde das Lösungsmittel entfernt. Ausbeute: quantitativ; Fp.: 93-94 °C.

Vorstufe 2β)

N-(4-Chlor-3-(1,3-dioxolan-2-yl)-phenylthiocarbamoyl]-1,4,5,6-tetrahydropyridazin

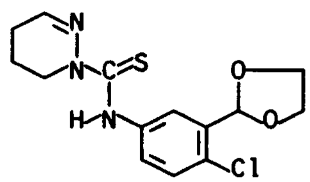

Bei 25 °C wurde eine Lösung der aus Vorstufe 2α) erhaltenen Rohsubstanz (ca. 0,5 mol 2-(1,3-Dioxolan-2-yl)-4-isothiocyanato-chlorbenzol) in 500 ml Tetrahydofuran zu einer Lösung von 42 g (0,5 mol) 1,4,5,6-Tetrahydropyridazin in 750 ml Tetrahydrofuran getropft. Nach 2 Stunden Rühren bei 25 °C wurde das Lösungsmittel entfernt und der Rückstand wurde mit 500 ml Ethanol verrührt. Der in der Lösung suspendierte Feststoff wurde abfiltriert und getrocknet. Ausbeute: 58 %; Fp.: 112-114 °C.

Vorstufe 2γ)

7-(2-Formyl-4-chlorphenylimino)-8-thia-1,6-diazabicyclo-[4.3.0]-non-2-en-9-on

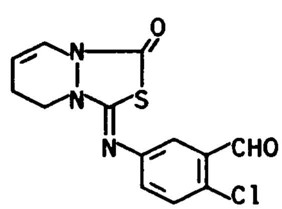

Zu einer Mischung aus 81,5 g (0,25 mol) N-[4-Chlor-3-(1,3-dioxolan-2-yl)-phenylthiocarbamoyl]-1,4,5,6-tetrahydropyridazin, 43 g (0,55 mol) Pyridin und 500 ml Methylenchlorid wurden bei 25-30 °C 50 g (0,25 mol) Chlorameisensäuretrichlormethylester in 200 ml Methylenchlorid getropft. Danach wurde 5 Stunden bei 25 °C gerührt und unter Eiskühlung 500 ml Wasser zugetropft. Nach Trennen der Phasen wurde die organische Phase neutral gewaschen und eingeengt. Den Rückstand hydrolysierte man 20 Stunden bei 40-45 °C mit 540 ml konz. Essigsäure. Anschließend versetzte man die Lösung bei 25 °C mit 300 ml Wasser. Der gebildete Feststoff wurde abfiltriert, mit Wasser neutral gewaschen und getrocknet.

Ausbeute: 61 %; Fp.: 145-146 °C.

Die physikalischen Daten der Endprodukte I sind der folgenden Tabelle 2 zu entnehmen, in der noch weitere Verbindungen I aufgeführt sind, welche auf die gleichen Weisen hergestellt wurden oder herstellbar

sind.

Tabelle 2

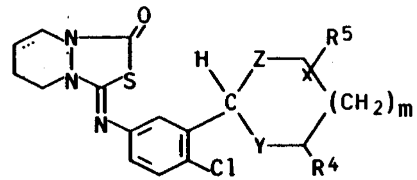

I ($R^1$, $R^3$=H; $R^2$=Cl; X=O)

| Nr. | Doppelbindung im Thia-diaza-bicyclononan-Teil | Y | Z | $R^4$ | $R^5$ | m | physik. Daten Fp [°C]; IR [$cm^{-1}$]; NMR [ppm] |
|---|---|---|---|---|---|---|---|
| 3.01 | ja | S | S | H | H | 1 | 180-183 |
| 3.02 | ja | O | O | H | H | 0 | 1695, 1627, 1290, 1262 |
| 3.03 | ja | O | O | H | H | 1 | 129-130 |
| 3.04 | ja | S | S | H | H | 0 | 139-140 |
| 3.05 | ja | S | S | $CH_3$ | H | 0 | 1694, 1626, 1584, 1288, 1266 |
| 3.06 | ja | O | S | H | H | 0 | 1691, 1618, 1586, 1262 |
| 3.07 | ja | O | O | $CH_3$ | 6-$CH_3$ | 1 | 1694, 1630, 1592, 1263 |
| 3.08 | ja | O | O | $CH_3$ | 5-$CH_3$ | 0 | 1696, 1628, 1262, 1089 |
| 3.09 | ja | O | O | $CH_3$ | 5-CO-O$(CH_2)_3CH_3$ | 0 | 1731, 1697, 1628, 1266 |
| 3.10 | ja | O | O | $CH_3$ | 5-CO-O$(CH_2)_3CH_3$ | 0 | |
| 3.11 | ja | O | O | H | 5-$CH_2OCH_2CH=CH_2$ | 0 | 1696, 1627, 1263, 1093 |
| 3.12 | nein | S | S | $CH_3$ | H | 0 | 121-122 |

Anwendungsbeispiele

Die herbizide Wirkung der Thiadiaza-bicyclononan-Derivate der Formel I ließ sich durch Gewächshausversuche zeigen:

Als Kulturgefäße dienten Plastikblumentöpfe mit 300 $cm^3$ Inhalt und lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 0,03 kg/ha a.S.

Die Pflanzen wurden artenspezifisch bei Temperaturen von 10-25 °C bzw. 20-35 °C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Botanischer Name | Deutscher Name |
|---|---|
| Triticum aestivum | Sommerweizen |
| Cassia tora | - |
| Chenopodium album | Weißer Gänsefuß |
| Ipomoea spp. | Prunkwindearten |

Das Ergebnis zeigte, daß mit der Verbindung Nr. 3.12 die breitblättrigen Pflanzen im Nachauflaufverfahren völlig vernichtet wurden. Die Kultur Weizen wurde dagegen nicht geschädigt.

**Patentansprüche**

**Patentansprüche für folgende Vertragsstaaten : BE, CH, DE, FR, GB, IT, LI, NL**

**1.** Thiadiaza-bicyclononan-Derivate der allgemeinen Formel I

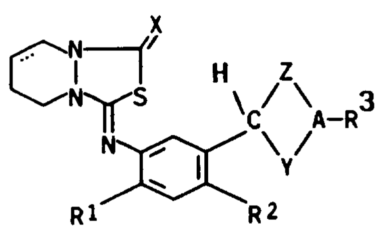

I

in der die punktierte Bindung für eine mögliche zusätzliche Bindung steht und die Substituenten folgende Bedeutung haben:

- $R^1$ Wasserstoff oder Fluor;
- $R^2$ Halogen;
- $R^3$ Wasserstoff, eine $C_1$-$C_8$-Alkoxycarbonylgruppe, eine $C_3$-$C_6$-Alkenyloxycarbonylgruppe oder eine $C_3$-$C_6$-Alkinyloxycarbonylgruppe, wobei diese 3 Gruppen jeweils noch einen $C_1$-$C_4$-Alkoxyrest tragen können;
- X,Y,Z Sauerstoff oder Schwefel;
- A eine $C_2$-$C_3$-Alkylenkette, welche zusätzlich zu $R^3$ bis zu drei gleiche oder verschiedene Reste $R^4$ tragen kann: Hydroxyl, Carboxyl, $C_1$-$C_4$-Alkyl oder $C_1$-$C_6$-Alkoxycarbonyl, wobei die beiden letztgenannten Reste ihrerseits bis zu 5 Halogenatome und einen der folgenden Substituenten tragen können:

Hydroxyl, Cyan, Mercapto, $C_1$-$C_4$-Alkoxy, $C_1$-$C_6$-Alkylthio, $C_3$-$C_6$-Alkenyloxy, $C_3$-$C_6$-Alkenylthio, $C_3$-$C_6$-Alkinyloxy, $C_3$-$C_6$-Alkinylthio, $C_1$-$C_6$-Alkylcarbonyloxy, $C_1$-$C_6$-Alkoxycarbonyl, $C_1$-$C_6$-Alkoxycarbonyl-$C_1$-$C_4$-alkoxy oder $C_1$-$C_6$-Alkoxycarbonyl-$C_1$-$C_4$-allkylthio.

**2.** Verfahren zur Herstellung der Verbindungen I gemäß Anspruch 1, dadurch gekennzeichnet, daß man

a) ein Nitrobenzol der allgemeinen Formel II

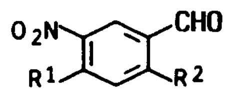

II

in an sich bekannter Weise mit einer Verbindung der allgemeinen Formel IIIa

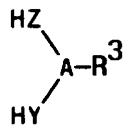

IIIa

acetalisiert,

b) das so erhaltene Acetal bzw. Thioacetal IV

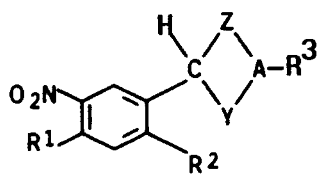

IV

zu einem Anilinderivat V

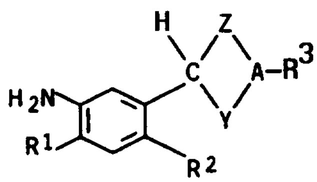

V

reduziert,

c) das so erhaltene Zwischenprodukt V mit Thiophosgen zu einem Isocyanat VI

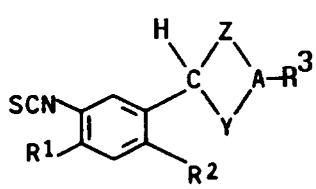

VI

umsetzt,

d) an die Verbindung VI anschließend ein cyclisches Hydrazin der allgemeinen Formel VII

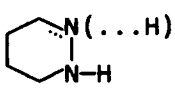

VII

wobei das eine Stickstoffatom entweder an einen Ringkohlenstoff doppelt gebunden ist oder wie das andere ein Wasserstoffatom trägt, addiert und

e) das erhaltene Produkt VIII

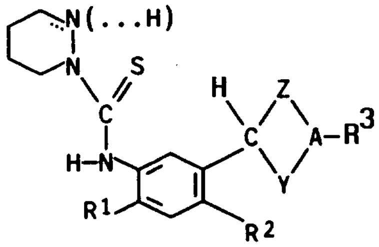

VIII

anschließend mit Phosgen, Chlorameisensäuretrichlormethylester oder Thiophosgen cyclisiert, oder

f) für den Fall, daß $R^3$ Wasserstoff und A eine Ethylen- oder Propylenkette, die jeweils noch bis zu 3 $C_1$-$C_3$-Alkylgruppen tragen kann, bedeutet, die Acetalgruppe im sauren Medium spaltet und die entstehende Carbonylverbindung IX

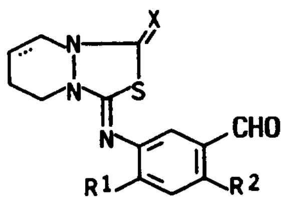

IX

abschließend mit einer Verbindung der Formel IIIb

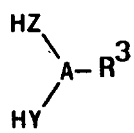

IIIb

acetalisiert.

**3.** Herbizide Mittel, enthaltend ein Acetal der Formel I gemäß Anspruch 1 und übliche Zusatzstoffe.

**4.** Herbizide Mittel gemäß Anspruch 3, enthaltend ein Acetal der Formel I und weitere wirksame Bestandteile.

**5.** Verfahren zur Bekämpfung unerwünschten Pflanzenwuchses, dadurch gekennzeichnet, daß man eine herbizide Menge eines Acetals der Formel I gemäß Anspruch 1 auf Pflanzen, deren Lebensraum oder auf Saatgut einwirken läßt.

**6.** Verwendung der Acetale I gemäß Anspruch 1 als Herbizide.

**7.** Thiadiaza-bicyclononan-Derivate I nach Anspruch 1, wobei $R^1$ Wasserstoff, $R^2$ Chlor und X Sauerstoff bedeuten.

**Patentansprüche für folgenden Vertragsstaat : ES**

**1.** Verfahren zur Herstellung von Thiadiaza-bicyclononan-Derivaten der allgemeinen Formel I

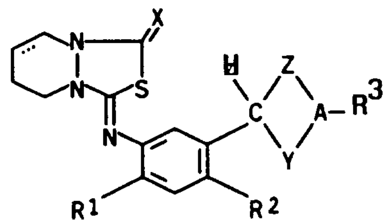

I

in der die punktierte Bindung für eine mögliche zusätzliche Bindung steht und die Substituenten folgende Bedeutung haben:

$R^1$ Wasserstoff oder Fluor;

$R^2$ Halogen;

$R^3$ Wasserstoff, eine $C_1$-$C_8$-Alkoxycarbonylgruppe, eine $C_3$-$C_6$-Alkenyloxycarbonylgruppe oder eine $C_3$-$C_6$-Alkinyloxycarbonylgruppe, wobei diese 3 Gruppen jeweils noch einen $C_1$-$C_4$-Alkoxyrest tragen können;

X,Y,Z Sauerstoff oder Schwefel;

A eine $C_2$-$C_3$-Alkylenkette, welche zusätzlich zu $R^3$ bis zu drei gleiche oder verschiedene Reste $R^4$ tragen kann: Hydroxyl, Carboxyl, $C_1$-$C_4$-Alkyl oder $C_1$-$C_6$-Alkoxycarbonyl, wobei die beiden letztgenannten Reste ihrerseits bis zu 5 Halogenatome und einen der folgenden Substituenten tragen können: Hydroxyl, Cyan, Mercapto, $C_1$-$C_4$-Alkoxy, $C_1$-$C_6$-Alkylthio, $C_3$-$C_6$-Alkenyloxy, $C_3$-$C_6$-Alkenylthio, $C_3$-$C_6$-Alkinyloxy, $C_3$-$C_6$-Alkinylthio, $C_1$-$C_6$-Alkylcarbonyloxy, $C_1$-$C_6$-Alkoxycarbonyl, $C_1$-$C_6$-Alkoxycarbonyl-$C_1$-$C_4$-alkoxy oder $C_1$-$C_6$-Alkoxycarbonyl-$C_1$-$C_4$-alkylthio,

dadurch gekennzeichnet, daß man

a) ein Nitrobenzol der allgemeinen Formel II

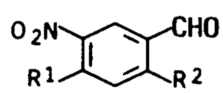

II

in an sich bekannter Weise mit einer Verbindung der allgemeinen Formel IIIa

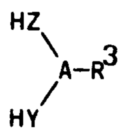

IIIa

acetalisiert,

b) das so erhaltene Acetal bzw. Thioacetal IV

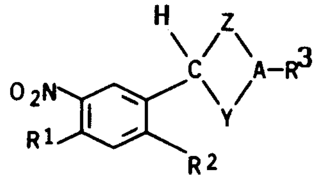

IV

zu einem Anilinderivat V

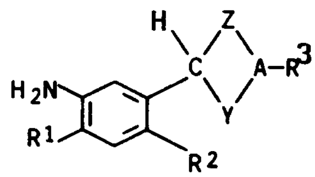

V

reduziert,

c) das so erhaltene Zwischenprodukt V mit Thiophosgen zu einem Isocyanat VI

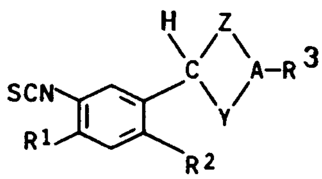

VI

umsetzt,

d) an die Verbindung VI anschließend ein cyclisches Hydrazin der allgemeinen Formel VII

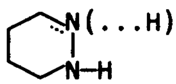

VII

wobei das eine Stickstoffatom entweder an einen Ringkohlenstoff doppelt gebunden ist oder wie das andere ein Wasserstoffatom trägt, addiert und

e) das erhaltene Produkt VIII

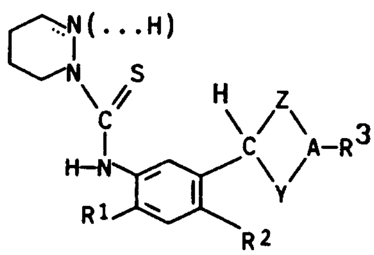

VIII

anschließend mit Phosgen, Chlorameisensäuretrichlormethylester oder Thiophosgen cyclisiert, oder

f) für den Fall, daß $R^3$ Wasserstoff und A eine Ethylen- oder Propylenkette, die jeweils noch bis zu 3 $C_1$-$C_3$-Alkylgruppen tragen kann, bedeutet, die Acetalgruppe im sauren Medium spaltet und die entstehende Carbonylverbindung IX

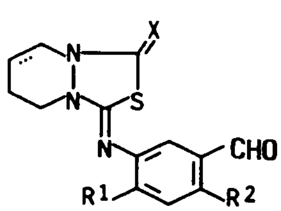

IX

abschließend mit einer Verbindung der Formel IIIb

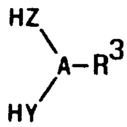

IIb

acetalisiert.

2. Verfahren nach Anspruch 1, dadurch gekennzeichnet, daß man es auf die Herstellung von Thiadiazabicyclononan-Derivaten I, wobei $R^1$ Wasserstoff, $R^2$ Chlor und X Sauerstoff bedeuten, anwendet.

3. Herbizide Mittel, enthaltend ein Acetal der Formel I gemäß Anspruch 1 und übliche Zusatzstoffe.

4. Herbizide Mittel gemäß Anspruch 3, enthaltend ein Acetal der Formel I und weitere wirksame Bestandteile.

5. Verfahren zur Bekämpfung unerwünschten Pflanzenwuchses, dadurch gekennzeichnet, daß man eine herbizide Menge eines Acetals der Formel I gemäß Anspruch 1 auf Pflanzen, deren Lebensraum oder auf Saatgut einwirken läßt.

6. Verwendung der Acetale I gemäß Anspruch 1 als Herbizide.

**Claims**
**Claims for the following Contracting States : BE, CH, DE, FR, GB, IT, LI, NL**

1. A thiadiazabicyclonane derivative of the general formula I

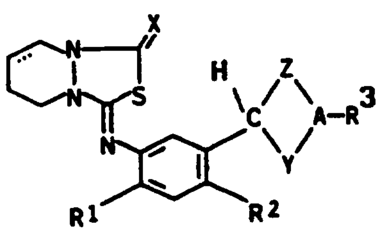

I

where the dotted bond is a possible additional bond and the substituents have the following meanings:

| | |
|---|---|
| $R^1$ | is hydrogen or fluorine; |
| $R^2$ | is halogen; |
| $R^3$ | is hydrogen, $C_1$-$C_8$-alkoxycarbonyl, $C_3$-$C_6$-alkenyloxycarbonyl or $C_3$-$C_6$-alkynyloxycarbonyl, it being possible for each of these 3 groups to additionally carry a $C_1$-$C_4$-alkoxy radical; |
| X, Y and Z | are oxygen or sulfur; |
| A | is a $C_2$-$C_3$-alkylene chain which, in addition to $R^3$, may carry up to three identical or different radicals $R^4$: hydroxyl, carboxyl, $C_1$-$C_4$-alkyl or $C_1$-$C_6$-alkoxycarbonyl, it being possible for the two last-mentioned radicals themselves to carry up to 5 halogen atoms and one of the following substituents: hydroxyl, cyano, mercapto, $C_1$-$C_4$-alkoxy, $C_1$-$C_6$-alkylthio, $C_3$-$C_6$-alkenyloxy, $C_3$-$C_6$-alkenylthio, $C_3$-$C_6$-alkynyloxy, $C_3$-$C_6$-alkynylthio, $C_1$-$C_6$-alkylcarbonyloxy, $C_1$-$C_6$-alkoxycarbonyl, $C_1$-$C_6$-alkoxycarbonyl-$C_1$-$C_4$-alkoxy or $C_1$-$C_6$-alkoxycarbonyl-$C_1$-$C_4$-alkylthio. |

2. A process for the preparation of a compound I as claimed in claim 1, which comprises
a) acetalating a nitrobenzene of the formula II

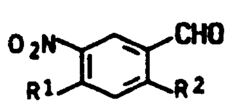

II

in a manner known per se with a compound of the formula IIIa

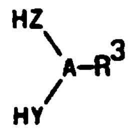

IIIa

b) reducing the resultant acetal or thioacetal IV

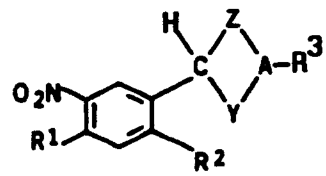

IV

to give an aniline derivative V

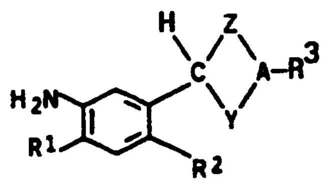

V

c) reacting the resultant intermediate V with thiophosgene to give an isocyanate VI

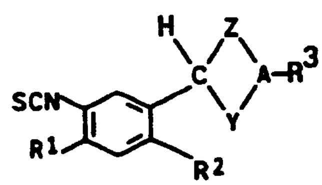

VI

d) subsequently adducting a cyclic hydrazine of the formula VII

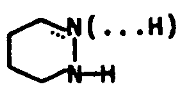

VII

where one nitrogen atom is either double-bonded to a ring carbon atom or, like the other, carries a hydrogen atom, onto the compound VI, and
e) subsequently cyclizing the resultant product VIII

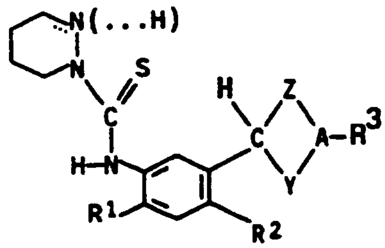

VIII

using phosgene, trichloromethyl chloroformate or thiophosgene, or

f) in the case where $R^3$ is hydrogen and A is an ethylene or propylene chain, each of which may also carry up to 3 $C_1$-$C_3$-alkyl groups, cleaving the acetal group in an acidic medium and subsequently acetalating the resultant carbonyl compound IX

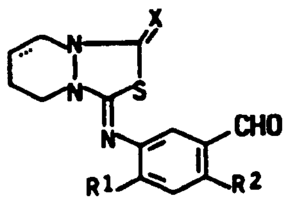

IX

with a compound of formula IIIb

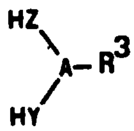

IIIb

**3.** A herbicide containing an acetal of the formula I as claimed in claim 1 and conventional additives.

**4.** A herbicide as claimed in claim 3, containing an acetal of the formula I and further active constituents.

**5.** A method for controlling unwanted plant growth, wherein a herbicidal amount of an acetal of the formula I as claimed in claim 1 is allowed to act on plants, their habitat or on seed.

**6.** The use of an acetal I as claimed in claim 1 as a herbicide.

**7.** A thiadiazabicyclononane derivative I as claimed in claim 1, where $R^1$ is hydrogen, $R^2$ is chlorine and X is oxygen.

**Claims for the following Contracting State : ES**

**1.** A process for the preparation of a thiadiazabicyclononane derivative of the general formula I

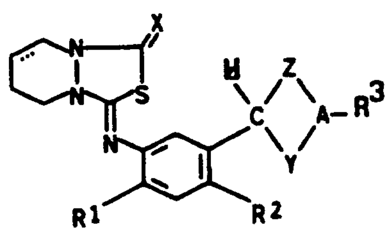

I

where the dotted bond is a possible additional bond and the substituents have the following meanings:

$R^1$ is hydrogen or fluorine;

$R^2$ is halogen;

$R^3$ is hydrogen, $C_1$-$C_8$-alkoxycarbonyl, $C_3$-$C_6$-alkenyloxycarbonyl or $C_3$-C6-alkynyloxycarbonyl, it being possible for each of these 3 groups to additionally carry a $C_1$-$C_4$-alkoxy radical;

X, Y and Z are oxygen or sulfur;

A is a $C_2$-$C_3$-alkylene chain which, in addition to $R^3$, may carry up to three identical or different radicals $R^4$: hydroxyl, carboxyl, $C_1$-$C_4$-alkyl or $C_1$-$C_6$-alkoxycarbonyl, it being possible for the two last-mentioned radicals themselves to carry up to 5 halogen atoms and one of the following substituents: hydroxyl, cyano, mercapto, $C_1$-$C_4$-

alkoxy, $C_1$-$C_6$-alkylthio, $C_3$-$C_6$-alkenyloxy, $C_3$-$C_6$-alkenylthio, $C_3$-$C_6$-alkynyloxy, $C_3$-$C_6$-alkynylthio, $C_1$-$C_6$-alkylcarbonyloxy, $C_1$-$C_6$-alkoxycarbonyl, $C_1$-$C_6$-alkoxycarbonyl-$C_1$-$C_4$-alkoxy or $C_1$-$C_6$-alkoxycarbonyl-$C_1$-$C_4$-alkylthio,

a) acetalating a nitrobenzene of the formula II

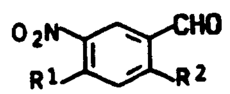

II

in a manner known per se with a compound of the formula IIIa

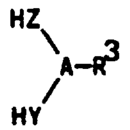

IIIa

b) reducing the resultant acetal or thioacetal IV

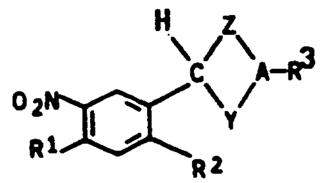

IV

to give an aniline derivative V

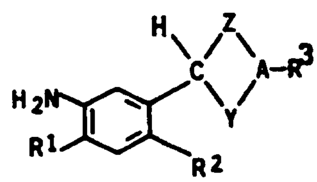

V

c) reacting the resultant intermediate V with thiophosgene to give an isocyanate VI

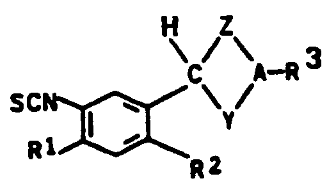

VI

d) subsequently adducting a cyclic hydrazine of the formula VII

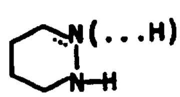

VII

where one nitrogen atom is either double-bonded to a ring carbon atom or, like the other, carries a hydrogen atom, onto the compound VI, and

e) subsequently cyclizing the resultant product VIII

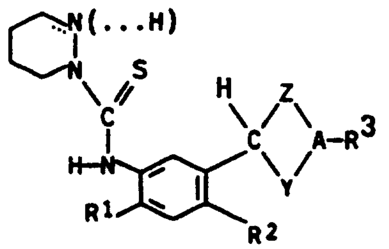

VIII

using phosgene, trichloromethyl chloroformate or thiophosgene, or

f) in the case where $R^3$ is hydrogen and A is an ethylene or propylene chain, each of which may also carry up to 3 $C_1$-$C_3$-alkyl groups, cleaving the acetal group in an acidic medium and subsequently acetalating the resultant carbonyl compound IX

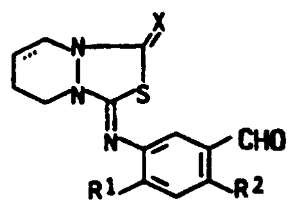

IX

with a compound of formula IIIb

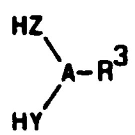

IIIb

2. A process as claimed in claim 1, which is used for the preparation of a thiadiazabicyclononane derivative I where $R^1$ is hydrogen, $R^2$ is chlorine and X is oxygen.

3. A herbicide containing an acetal of the formula I as claimed in claim 1 and conventional additives.

4. A herbicide as claimed in claim 3, containing an acetal of the formula I and further active constituents.

5. A method for controlling unwanted plant growth, wherein a herbicidal amount of an acetal of the formula I as claimed in claim 1 is allowed to act on plants, their habitat or on seed.

6. The use of an acetal I as claimed in claim 1 as a herbicide.

**Revendications**

**Revendications pour les Etats contractants suivants : BE, CH, DE, FR, GB, IT, LI, NL**

1. Dérivés de thiadiaza-bicyclononane de formule générale I

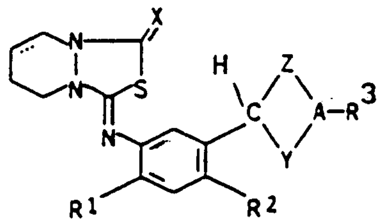

I

où la liaison en pointillé désigne une liaison additionnelle éventuelle et les substituants ont la signification suivante:

| | |
|---|---|
| $R^1$ | hydrogène ou fluor; |
| $R^2$ | halogène; |
| $R^3$ | hydrogène, un groupe alcoxy($C_1$-$C_8$)carbonyle, un groupe alcényloxy($C_3$-$C_6$)carbonyle ou un groupe alcynyloxy($C_3$-$C_6$)carbonyle, tandis que ces 3 groupes peuvent porter chacun encore un reste alcoxy en $C_1$-$C_4$; |
| X, Y, Z | oxygène ou soufre; |
| A | une chaîne alkylène en $C_2$-$C_3$, qui peut porter en plus de $R^3$ un à trois restes $R^4$ identiques ou différents: hydroxyle, carboxyle, alkyle en $C_1$-$C_4$ ou alcoxy($C_1$-$C_6$)carbonyle, ces deux restes nommés en dernier pouvant porter à leur tour jusqu'à 5 atomes d'halogène et l'un des substituants suivants: hydroxyle, cyano, mercapto, alcoxy en $C_1$-$C_4$, alkylthio en $C_1$-$C_6$, alcényloxy en $C_3$-$C_6$, alcénylthio en $C_3$-$C_6$, alcynyloxy en $C_3$-$C_6$, alcynylthio en $C_3$-$C_6$, alkyl($C_1$-$C_6$)-carbonyloxy, alcoxy($C_1$-$C_6$)carbonyle, alcoxy($C_1$-$C_6$)carbonylalcoxy en $C_1$-$C_4$ ou alcoxy-($C_1$-$C_6$)carbonylalkylthio en $C_1$-$C_4$. |

2. Procédé pour la préparation des composés I selon la revendication 1, caractérisé en ce que

a) l'on acétalise un nitrobenzène de formule générale II

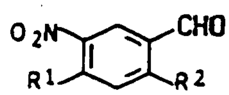

II

de manière connue en soi avec un composé de formule générale IIIa

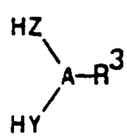

IIIa

b) l'on réduit l'acétal ou thioacétal IV ainsi obtenu

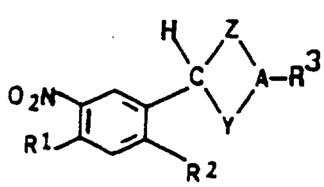

IV

en un dérivé d'aniline V

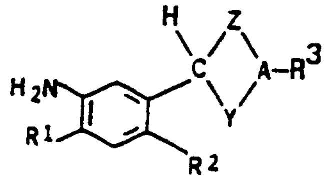

V

c) l'on convertit le produit intermédiaire V ainsi obtenu avec du thiophosgène en un isocyanate VI

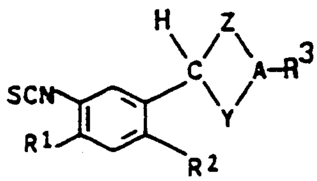

VI

d) l'on ajoute ensuite au composé VI une hydrazine cyclique de formule générale VII

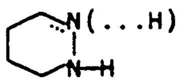

VII

où l'un des atomes d'azote soit est relié par une liaison double à un atome de carbone du cycle, soit porte comme l'autre un atome d'hydrogène, et

e) l'on cyclise le produit VIII obtenu

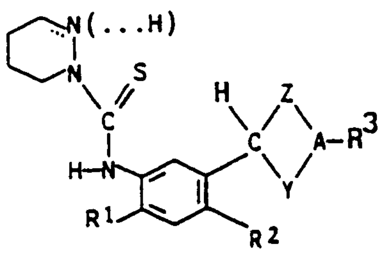

VIII

avec du phosgène, de l'ester trichlorométhylique d'acide chloroformique ou du thiophosgène,

ou

f) dans le cas où $R^3$ désigne l'hydrogène et A représente une chaîne éthylénique ou propylénique, qui peut porter dans chaque cas jusqu'à 3 groupes alkyle en $C_1$-$C_3$, l'on ouvre le groupe acétal en milieu acide et enfin l'on acétalise le composé carbonylé IX correspondant

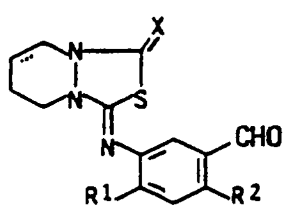

IX

avec un composé de formule IIIb

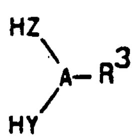

IIIb

**3.** Agents herbicides, contenant un acétal de formule I selon la revendication 1 et des additifs classiques.

**4.** Agents herbicides selon la revendication 3, contenant un acétal de formule I et d'autres constituants actifs.

**5.** Procédé pour lutter contre la croissance des plantes parasites, caractérisé en ce qu'on fait agir une quantité herbicide d'un acétal de formule I selon la revendication 1 sur les plantes, leur biotope ou leurs semences.

**6.** Utilisation des acétals I selon la revendication 1 comme herbicides.

**7.** Dérivés de thiadiaza-bicyclononane I selon la revendication 1, dans lesquels $R^1$ représente l'hydrogène, $R^2$ désigne le chlore et X désigne l'oxygène.

**Revendications pour l'Etat contractant suivant : ES**

**1.** Procédé pour la préparation de dérivés de thiadiaza-bicyclononane de formule générale I

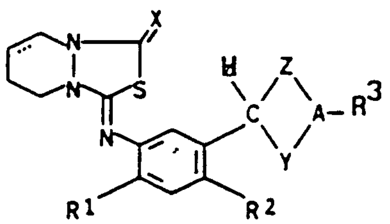

I

où la liaison en pointillé désigne une liaison additionnelle éventuelle et les substituants ont la signification suivante:

$R^1$ hydrogène ou fluor;

$R^2$ halogène;

$R^3$ hydrogène, un groupe alcoxy($C_1$-$C_8$)carbonyle, un groupe alcényloxy($C_3$-$C_6$)carbonyle ou un groupe alcynyloxy($C_3$-$C_6$)carbonyle, tandis que ces 3 groupes peuvent porter chacun encore un reste alcoxy en $C_1$-$C_4$;

X, Y, Z oxygène ou soufre;

A une chaîne alkylène en $C_2$-$C_3$, qui peut porter en plus de $R^3$ un à trois restes $R^4$ identiques ou différents: hydroxyle, carboxyle,
alkyle en $C_1$-$C_4$ ou alcoxy($C_1$-$C_6$)carbonyle, ces deux restes nommés en dernier pouvant porter à leur tour jusqu'à 5 atomes d'halogène et l'un des substituants suivants: hydroxyle, cyano, mercapto, alcoxy en $C_1$-$C_4$, alkylthio en $C_1$-$C_6$, alcényloxy en $C_3$-$C_6$, alcénylthio en $C_3$-$C_6$, alcynyloxy en $C_3$-$C_6$, alcynylthio en $C_3$-$C_6$, alkyl($C_1$-$C_6$)-carbonyloxy, alcoxy($C_1$-$C_6$)carbonyle, alcoxy($C_1$-$C_6$)carbonylalcoxy en $C_1$-$C_4$ ou alcoxy-($C_1$-$C_6$)carbonylalkylthio en $C_1$-$C_4$,

caractérisé en ce que

a) l'on acétalise un nitrobenzène de formule générale II

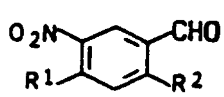

II

de manière connue en soi avec un composé de formule générale IIIa

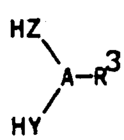

IIIa

b) l'on réduit l'acétal ou thioacétal IV ainsi obtenu

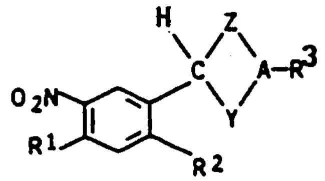

IV

en un dérivé d'aniline V

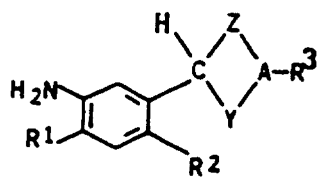

V

c) l'on convertit le produit intermédiaire V ainsi obtenu avec du thiophosgène en un isocyanate VI

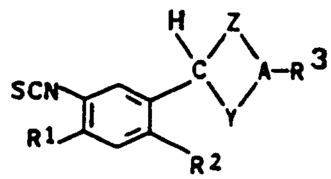

VI

d) l'on ajoute ensuite au composé VI une hydrazine cyclique de formule générale VII

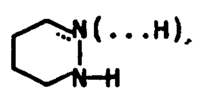

VII

où l'un des atomes d'azote soit, est relié par une liaison double à un atome de carbone du cycle, soit porte comme l'autre un atome d'hydrogène, et

e) l'on cyclise le produit VIII obtenu

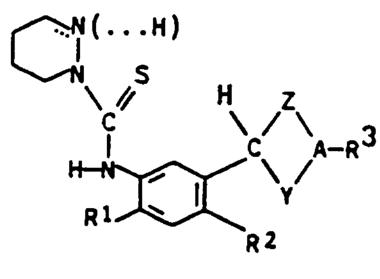

VIII

avec du phosgène, de l'ester trichlorométhylique d'acide chloroformique ou du thiophosgène,

ou

f) dans le cas où $R^3$ désigne l'hydrogène et A représente une chaîne éthylénique ou propylénique, qui peut porter dans chaque cas jusqu'à 3 groupes alkyle en $C_1$-$C_3$, l'on ouvre le groupe acétal en milieu acide et enfin l'on acétalise le composé carbonylé IX correspondant

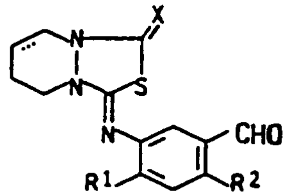

IX

avec un composé de formule IIIb

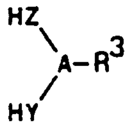

IIIb

2. Procéde selon la revendication 1, caractérisé en ce qu'on l'utilise pour la préparation de dérivés de thiadiaza-bicyclononane I, où $R^1$ représente l'hydrogène, $R^2$ désigne le chlore et X désigne l'oxygène.

3. Agents herbicides, contenant un acétal de formule I selon la revendication 1 et des additifs classiques.

4. Agents herbicides selon la revendication 3, contenant un acétal de formule I et d'autres constituants actifs.

5. Procédé pour lutter contre la croissance des plantes parasites, caractérisé en ce qu'on fait agir une quantité herbicide d'un acétal de formule I selon la revendication 1 sur des plantes, leur biotope ou leurs semences.

6. Utilisation des acétals I selon la revendication 1 comme herbicides.